(19) Europäisches Patentamt
European Patent Office
Office européen des brevets

(11) Veröffentlichungsnummer: **0 345 583**
**A2**

# (12) EUROPÄISCHE PATENTANMELDUNG

(21) Anmeldenummer: **89109613.3**

(22) Anmeldetag: **27.05.89**

(51) Int. Cl.4: **C07H 15/04 , A61K 31/70**

(30) Priorität: **09.06.88 DE 3819634**

(43) Veröffentlichungstag der Anmeldung:
**13.12.89 Patentblatt 89/50**

(84) Benannte Vertragsstaaten:
**AT BE CH DE ES FR GB GR IT LI LU NL SE**

(71) Anmelder: **BAYER AG**
**D-5090 Leverkusen 1 Bayerwerk(DE)**

(72) Erfinder: **Tietze, Lutz-Friedjahn, Prof. Dr.**
**Stumpfe Eiche 73**
**D-3400 Goettingen(DE)**
Erfinder: **Neumann, Manfred, Dr.**
**Muensterlandstrasse 18**
**D-4370 Marl(DE)**
Erfinder: **Fischer, Roland, Dr.**
**Steffensweg 85**
**D-3400 Goettingen(DE)**

## (54) Neue Aldophosphamidglycoside, Verfahren zu ihrer Herstellung sowie ihre Verwendung als pharmazeutische Wirkstoffe.

(57) Die Erfindung betrifft neue säurelabile Acetalglycoside von Aldophosphamid und anderen cytociden Aldehyden als Acetalkomponente, ein Verfahren zu ihrer Herstellung und ihre Verwendung als hochselektive Cytostatika in der Krebstherapie.

EP 0 345 583 A2

# Neue Aldophophamidglycoside, Verfahren zu ihrer Herstellung sowie ihre Verwendung als pharmazeutische Wirkstoffe

Die Erfindung betrifft neue säurelabile Acetalglycoside von Aldophosphamid und anderen cytociden Aldehyden als Acetalkomponente, ein Verfahren zu ihrer Herstellung und ihre Verwendung als hochselektive Cytostatika in der Krebstherapie.

Es ist bekannt, daß maligne Zellen eine gegenüber dem Normalgewebe erhöhte Glycolyse und damit Lactatproduktion aufzeigen und der pH-Wert im Tumorgewebe durch intravenös verabreichte Glucose gesenkt werden kann (vgl. S. Tanneberger, Experimentelle und klinische Tumorchemotherapie; Allgemeine Tumorchemotherapie; G. Fischer Verlag, Stuttgart/New York 1980; Naturwiss. 46, 2(1959); Cancer Res. 42, 1498 (1982) ; 42, 1505 (1982)).

In der Vergangenheit wurde versucht, diese Unterschiede im pH-Wert zwischen normalem und Tumorgewebe für eine selektive Tumortherapie auszunutzen (vergl. Liebigs Ann. Chem. 1987, 847-856; Tetrahedron Lett. 22 (1981) 239-242). Es war angestrebt worden, alkylierende Verbindun gen, die wegen einer zu geringen Differenzierung zwischen gesundem und malignem Gewebe eine sehr geringe therapeutische Breite aufweisen, in untoxische, säurelabile Prodrug-Formen zu überführen, die nur im Tumorgewebe aufgrund des dort vorherrschenden erniedrigten pH-Wert selektiv gespalten werden zum aktiven, alkylierend wirkenden Cytostatikum. Auf diese Weise sollte eine selektive Tumortherapie angestrebt werden.

Es hatte sich aber gezeigt, daß die in der oben angeführten Literaturstelle hergestellten Verbindungen sich nicht als so säurelabil erwiesen, als daß sie selektiv im Tumorgewebe zum aktiven cytociden Agenz rückgespalten wurden.

Es wurde nun überraschenderweise festgestellt, daß die folgenden genannten Verbindungen untoxisch sind, während sie bei dem in Tumorgewebe durch Hyperglykämie erreichbaren pH-Wert durch Hydrolyse in cytocide Verbindungen überführt werden können.

Die neuen Verbindungen entsprechen der allgemeinen Formel (I), (II) und (III)

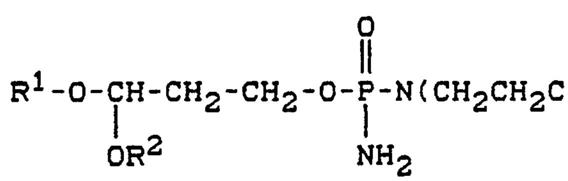
(I)

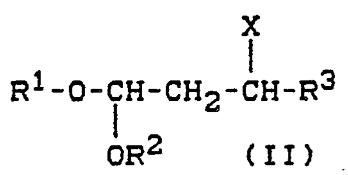
(II)

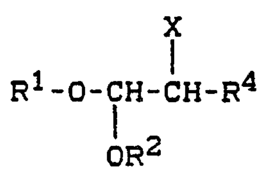
(III)

in welcher

$R^1$ für gesättigtes, ungesättigtes oder anhydridisches Desoxypentosyl, Desoxyhexosyl oder Didesoxyhexosyl steht,

wobei

die OH-Funktionen des Zuckers gegebenenfalls geschützt sind,

und

$R^2$ für geradkettiges oder verzweigtes auch heterosubstituiertes Alkyl mit bis zu zehn Kohlenstoffatomen steht und

$R^3$ für geradkettiges oder verzweigtes auch heterosubstituiertes Alkyl mit bis zu zehn Kohlenstoffatomen und

X für Halogene und

$R^4$ für geradkettiges oder verzweigtes, auch heterosubstituiertes Alkyl mit bis zu zehn Kohlenstoffatomen steht.

Bevorzugt sind solche Verbindungen der allgemeinen Formel (I), (II) und (III)

in welcher

$R^1$ für gesättigtes, ungesättigtes oder anhydridisches 2-Desoxyhexopyranosyl, 2,6-Didesoxy-hexopyranosyl, 2-Desoxy-pentofuranosyl, 2,3-Didesoxyhex-enopyranosyl oder 2,3-Anhydropentofuranosyl steht,

wobei
die OH-Funktionen des Zuckers gegebenenfalls geschützt sind,
und
$R^2$ für geradkettiges oder verzweigtes Alkyl mit bis zu 6 Kohlenstoffatomen und
$R^3$ und $R^4$ für Wasserstoffatomen steht.

Besonders bevorzugt sind die Verbindungen der allgemeinen Formeln (I), (II) und (III)
in welcher
$R^1$ für 2-Desoxy-α-D-arabino-hexo-pyranosyl, 2-Desoxy-D-erythro-pento-pyranosyl, 2,6-Didesoxy-α-L-arabino-hexo-pyranosyl, 2,3-Anhydro-α-D-manno-pyranosyl oder 2,3-Didesoxy-D-erythro-hex-2-eno-pyranosyl steht,
wobei
die OH-Funktionen des Zuckers gegebenenfalls geschützt sind und
$R^2$ für geradkettiges Alkyl mit bis zu 3 Kohlenstoffatomen,
$R^3$ und $R^4$ für Wasserstoffatome und
X für Halogen steht.

Ganz besonders bevorzugt sind die Verbindungen der allgemeinen Formeln (I), (II) und (III)
in welcher
$R^1$ für 2-Desoxy-α-D-arabino-hexo-pyranosyl, 2,6-Didesoxy-α-L-arabino-hexo-pyranosyl oder 2,3-Didesoxy-D-erythro-hex-2-eno-pyranosyl steht,
wobei
die OH-Funtionen des Zuckers gegebenenfalls geschützt sind
und
$R^2$ für Methyl oder Ethyl,
$R^3$ und $R^4$ für Wasserstoffatome und
X für Halogen steht.

Als OH-Schutzgruppen eignen sich die üblichen Schutzgruppen wie Methylbenzoyl, Benzoyl, Acetyl oder Benzyl. Bevorzugt sind Benzyl, Acetyl oder Methylbenzoyl. Besonders bevorzugt ist Acetyl (Ac).

Außerdem wurde ein Verfahren zur Herstellung der erfindungsgemäßen Verbindungen der allgemeinen Formel (I) gefunden,
dadurch gekennzeichnet,
daß man Verbindungen der allgemeinen Formel (IV)

$$R^1\text{-O-}\ \underset{\displaystyle \underset{|}{} \atop \displaystyle O\,R^2}{C}H\ \text{-}CH_2\text{-}CH_2\text{-}R^5 \qquad (IV)$$

in welcher
$R^1$ und $R^2$ die oben angegebene Bedeutung haben und
$R^5$ für Hydroxy steht
mit
N,N-Bis(2-chlorethyl)-phosphorsäuramid-dichlorid der Formel (V)

$Cl_2PON(CH_2CH_2Cl)_2$ (V)

in Anwesenheit von Basen umsetzt, nachfolgend eine Ammonolyse durchführt und gegebenenfalls die Schutzgruppen am Zuckerrest abspaltet.

N,N-Bis-(2-chorethyl)-phosphorsäureamiddichlorid der Formel (V) ist bekannt (vgl. J. Am. Chem. Soc. 76, 655 (1954)).

Für das Verfahren kommen als Verdünnungsmittel die üblichen inerten organischen Lösungsmittel in Frage, die sich unter den Reaktionsbedingungen nicht verändern. Hierzu gehören bevorzugt Benzol, Toluol, Xylol, Dichlormethan oder dimethylformamid. Ebenso ist es möglich, Gemische der genannten Lösungsmittel zu verwenden. Besonders bevorzugt setzt man Benzol und Dichlormethan im Verhältnis 1:1 ein.

Als Basen können tert. Amine verwendet werden. Besonders bevorzugt ist Triethylamin.

Die Reaktion verläuft in einem Temperaturbereich von 20 °C-100 °C, bevorzugt bei 40 °C.

Das Verfahren wird bei Normaldruck durchgeführt.

Die Ammonolyse erfolgt bevorzugt bei Raumtemperatur.

Die Verbindungen der Formel IV sind neu und können aus den Verbindungen der Formel (IVa)

$$R^1\text{-O-}\ \underset{\displaystyle \underset{|}{} \atop \displaystyle O\,R^2}{C}H\text{-}\ CH_2\text{-}CH_2\text{-}O\text{-}CH_2\text{-}R^4 \qquad (IVa)$$

in welcher
$R^1$ und $R^2$ die oben angegebene Bedeutung haben, und
$R^4$ für Phenyl oder Methoxyphenyl steht,
durch Debenzylierung nach bekannten Methoden erhalten werden (vgl. Reaktionen und Synthesen im

organisch-chemischen Praktikum, Thieme-Verlag, Stuttgart, New-York 1981).

Die Verbindungen der allgemeinen Formel (IVa) sind neu und können aus den Verbindungen der Formel (VI)

$R^1$-O-Si($CH_3$)$_3$ (VI)

durch Umsetzung mit Acetalen der Formel (VII)

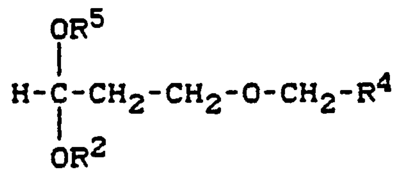

(VII)

in welcher

$R^2$ und $R^4$ die oben angegebene Bedeutung haben,

und

$R^5$ für Methyl oder Ethyl steht

und dem der Verbindung V zugrunde liegenden Aldehyd der Formel VIII

$O=HC\text{-}CH_2\text{-}CH_2\text{-}O\text{-}CH_2\text{-}R^4$ (VIII)

erhalten werden.

Die Verbindungen der Formel (VIII) können nach bekannten Verfahren aus den Desoxykohlenhydraten hergestellt werden (vgl. J. Org. Chem. 26, 908, 1961 und Can. J. Chem. 56, 2889, 1978).

Die Acetale der Formel (VII) sind bekannt (vgl. Heterocycles 15, 999 (1981); Chem. Pharm. Bull. 15, 1893 (1967)).

Ebenfalls bekannt ist das 3-Phenylmethoxypropionaldehyd (vgl. Synthesis, 1981, 165).

Die erfindungsgemäßen Verbindungen der allgemeinen Formel (I), (II) und (III) zeigen ein nicht vorhersehbares, wertvolles pharmakologisches Wirkspektrum.

Die säurelabilen Glycoside des inaktivierten Cyclophosphamids der allgemeinen Formel (I) stellen erstmalig ein Cytostatikum dar, das in seiner Transportform untoxisch ist. Bei dem im Tumorgewebe durch Hyperglykämie erreichbaren pH-Wert setzen sie durch Hydrolyse eine cytocide Verbindung, die Friedman-Säure frei. Spaltungsversuche, die nach der Arbeitsvorschrift 7 (AAV7) durchgeführt wurden, zeigen, daß bevorzugt (3'RS)-1'-O-(3'-Ethoxy-3'-(2,3-di-desoxy-D-erythro-hex-2-enopyranosyloxy))-propyl-N,N-bis-(2-chlorethyl)-phosphorsäurediamidat (Beispiel 20) bei pH = 5 innerhalb von 7 d vollständig, bei pH = 5,5 zu 80 % und bei pH = 6 zu 20 % gespalten wird.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein. d.h. in Mengen, die ausreichend sind, um den angegebenen Dosierungsspielraum zu speichern.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche öle (z.B. Erdnuß/Sesamöl), Alkohole, (z.B. Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether-Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den oben genannten Hilfsstoffen mit verschiedenen Geschmacksaufbesserern oder Farbstoffen versetzt werden.

Bei oraler Applikation sind solche galenischen Zubereitungen zu verwenden bei denen die Freisetzung der Wirkstoffe erst im Darm erfolgt.

Eine Freisetzung im Magen kann zu einer unerwünschten vorzeitigen Acidolyse der erfindungsgemäßen Substanzen führen.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Aplikation beträgt die Dosierung etwa 0,01 bis 20 mg/kg vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art der Applikation, aber auch aufgrund der Erkrankung und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen.

## Experimenteller Teil

### Allgemeine Arbeitsvorschriften

Allgemeine Arbeitsvorschrift 1 zur Herstellung von Trimethylsilylglycosiden der Formel (VI) aus den entsprechenden 1-Hydroxyverbindungen.

Man löste 50.0 mmol 1-Hydroxyverbindung in 50 ml absolutem Pyridin und kühlte unter Argon-Atmosphäre auf 0 °C ab. In die Lösung tropfte man 7.0 ml 1,1,1,3,3,3-Hexamethyldisilazan (33.0 mmol) und anschließend 2,2 ml Trimethylchlorsilan (17.0 mmol), wobei sich weißes Pyridiniumhydrochlorid abschied. Der Umsatz wurde dünnschichtchromatographisch verfolgt. Zur Aufarbeitung wurde das Pyridin bei 40 °C am Rotationsverdampfer entfernt und der weiße, ölige Rückstand in absolutem Ether aufgenommen. Die Suspension wurde über Celite säulenfiltriert, eingeengt und in Abhängigkeit von der Verbindung chromatographiert, destilliert oder kristallisiert.

Allgemeine Arbeitsvorschift 2 zur Herstellung der Acetalglycoside der Formel (IVa).

Eine Lösung von 1.00 mmol Trimethylsilylglycosid der Formel (VI) wurde unter Feuchtigkeitsausschluß in 5 ml absolutem Dichlormethan gelöst, mit 1.00 mmol Acetal der Formel (VII) und 0.50 mmol Aldehyd der Formel (VIII) versetzt. Man kühlte das Gemisch unter Argonatmosphäre in Abhängigkeit von den Reaktanten auf -50 bis -180 °C ab. 18 µl Trifluormethansulfonsäuretrimethylsilylester (0.10 mmol) wurden langsam mit einer Spritze zugetropft.

Nach beendeter Reaktion (DC-Kontrolle) versetzte man mit 28 µl Triethylamin (0.20 mmol) und ließ sich das Reaktionsgemisch auf 20 °C erwärmen. Danach wurde mit Dichlormethan über 2 g Kieselgel (60-200 µm) säulenfiltriert und das Eluat einrotiert. Reinigung und Diastereomerentrennung erfolgte durch Chromatographie an Kieselgel (32 - 63 µm).

Allgemeine Arbeitsvorschrift 3 (Variante zur Arbeitsvorschift 2)

Eine Lösung von 1.00 mmol Trimethylsilylglycosid der Formel (VI) wurde unter Feuchtigkeitsausschluß in 5 ml absolutem Dichlormethan gelöst, mit 1.50 mmol Acetal der Formel (VII) und 37 µl Aceton (0.50 mmol) versetzt. Man kühlte das Reaktionsgemisch unter Argonatmosphäre in Abhängigkeit von den Reaktanden auf -50 bis -78 °C ab. 18 µl Trifluormethansulfonsäuretrimethylsilylester (0.10 mmol) wurden langsam mit einer Spritze zugetropft. Nach beendeter Reaktion (DC-Kontrolle) versetzte man mit 28 µl Triethylamin (0.20 mmol) und ließ das Reaktionsgemisch auf 20 °C erwärmen. Danach wurde mit Dichlormethan über 2 g Kieselgel (60- 200 µm) säulenfiltriert und das Eluat einrotiert. Reinigung und Diastereomerentrennung erfolgte durch Chromatographie an Kieselgel (32 -63 µm).

Allgemeine Arbeitsvorschrift 4 (Hydrogenolyse der Acetalglycosidbenzylether der Formal (IVa) zu den Alkoholen der Formel (IV))

1.00 mmol der Benzylverbindung der Formel(IVa) wurde in einem Gemisch aus 15 ml Essigsäureethylester und 5 ml Ethanol gelöst.

212 mg Palladium auf Aktivkohle (= 0.20 mmol Pd) und 106 mg Palladium auf Calciumcarbonat (= 0.10 mmol Pd) wurden hinzugefügt und unter einer Wasserstoffatmosphäre bei 1 bar bei Raumtemperatur geschüttelt. Nach vollständigem Umsatz (DC-Kontrolle) wurde über eine Glasfritte (P3) vom Katalysator abfiltriert, eingeengt und an Kieselgel chromatographiert.

Allgemeine Arbeitsvorschrift 5 (Synthese der geschützten Aldophosphamidglycoside der Formel (I))

1.00 mmol der Hydroxyverbindung der Formel (IV) wurde in 4 ml eines Gemisches aus abs. Benzol und abs. Dichlormethan (1:1) unter Feuchtigkeitsausschluß gelöst. Man gab 558 µl absolutes Triethylamin (4.00 mmol) mit einer Spritze hinzu. 338 mg N,N-Bis(2-chlorethyl)phosphorsäuredichlorid (V) (1.50 mmol) wurden zugegeben und bei 40° C gerührt. Nach vollständiger Abreaktion des Eduktes (DC-Kontrolle) wurde bis zur Trockene einrotiert und in 5 ml abs. Toluol aufgenommen. Bei Raumtemperatur leitete man langsam getrocknetes Ammoniakgas ein. Den Ablauf der Reaktion überprüfte man dünnschichtchromatographisch und entfernte nach Beendigung das Toluol im Vakuum. Anschließend wurde an Kieselgel chromatographiert.

Allgemeine Arbeitsvorschrift 6 (Entblockierung der geschützten Acetalglycoside der Formel (I))

60 mg der zu entschützenden Verbindung der Formel (I) wurden in 1.5 ml abs. Methanol gelöst. Man gab 15 mg $K_2CO_3$ hinzu. Nach 45 min wurde mit 1.5 ml Ether versetzt und durch 5 g Kieselgel (60 -200 µm) (LS 8) filtriert.

Nach Entfernen des Lösungsmittels wurde die Verbindung für die Spaltung eingesetzt.

Allgemeine Arbeitsvorschrift 7 (Säurespaltung der Acetalglycoside der Formel (I))

Die zu spaltende Verbindung wurde in 1 ml Wasser (bidest.) gelöst. Je 200 µl wurden in 1 ml eines Puffersystems gegeben, das auf 37° C thermostatiert war. Bei folgenden pH-Werten wurden Versuche durchgeführt: 5.0, 5.5, 6.0, 6.5, 7.0. Die Spaltungen wurden nach 1h, 2h, 4h, 8h, 24h und danach im Abstand von 24h dünnschichtchromatographisch untersucht. Die Konzentration der benutzten Phosphatpuffersysteme betrug 0.05 mol/l.

Allgemeine Methoden

Dünnschichtchromatographie: DC Fertigfolien SIL G/$UV_{254}$'

Laufmittelsysteme:

1) Essigsäureethylester/Petrolether (40-60): a) 1:1, b) 1:2, c) 2:1, d) 3:1,
2) tert-Butylmethylether/Petrolether (40-60) a) 1:1, b) 1:2, c) 1:3, d) 1:4, e)2:3,
3) Aceton/Petrolether (40-60) a) 1:5, b) 3:2, c) 3:2,
4) Ether/Petrolether (40-60) a) 1:1, b) 1:2, c) 1:5,
5) Dichlormethan/Methanol/Petrolether (40-60) a) 10:1:1, b) 3:1:1,
6) tert-Butylmethylether/Dichlormethan/Petrolether (40-60) 1:10:3,

7) tert-Butylmethylether/Dichlormethan 1:4
8) Diethylether/Methanol 1:1

Beispiel 1

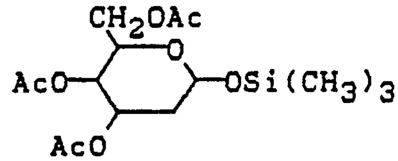

Synthese des Eduktes: Trimethylsilyl-3,4,6-tri-O-acetyl-2-desoxy-α-D-arabino-hexopyranosid

17.5 g 3,4,6-Tri-O-acetyl-2-desoxy-α-D-arabino-hexopyranose (60.3 mmol) wurden nach der AAV1 umgesetzt. Nach beendeter Reaktion (15 min) wurde aufgearbeitet und im Vakuum destilliert (Kugelrohr). Sdp. 180 °C/0.06 Torr. Man erhielt 20 g (92 %) einer farblosen Flüssigkeit, die nach wenigen Stunden kristallisierte.

Smp. 52 °C, $[\alpha]_D^{20}$ = + 102.8° (c = 0,7, $CHCl_3$).

$R_F$ = 0.60 (LS 1a).

IR (KBr): 2980 (C-H), 1745 (C=O), 1365 (C-H), 1250, 1230 (C-O), 1045, 1005, 840 $cm^{-1}$

$^1$H-NMR (200 MHz, $CDCl_3$): δ = 0.12 (s; 9H, $Si(CH_3)_3$), 1.79 (ddd, $J_{1,2a}$ = 3.0 Hz, $J_{2e,2a}$ = 12,5 Hz, $J_{2a,3}$ = 11,5 Hz, 1 H, 2a-H), 2.05, 2.02, 1,99 (3xs; 9H, OAc), 2.12 (ddd, $J_{2e,1}$ = 1,5 Hz, $J_{2e,2a}$ = 12,5 Hz, $J_{2,3}$ = 5,5 Hz; 1 H, 2-$H_e$), 3.99 (dd, $J_{6,6'}$ = 12,0 Hz, $J_{6,5}$ = 2,0 Hz; 1 H, 6-H), 4.09 (ddd, $J_{5,6}$ = 2,0 Hz, $J_{5,6'}$ = 4,5 Hz, $J_{5,4}$ = 10,0 Hz; 1 H, 5-H), 4.29 (dd, $J_{6',6}$ = 12.0 Hz, $J_{6',5}$ = 4,5 Hz; 1 H, 6'-H), 4.97 (t, $J_{4,5}$ = $J_{4,3}$ = 10,0 Hz; 1 H, 4-H) 5.34 (dd, $J_{1,2e}$ = 1,5 Hz, $J_{1,2a}$ = 3,0 Hz; 1 H, 1-H), 5,37 (ddd, $J_{3,2e}$ = 5,5 Hz, $J_{3,2a}$ = 11,5 Hz; $J_{3,4}$ = 10 Hz; 1 H, 3-H)

$^{13}$C-NMR (50.3 MHz, $CDCl_3$): δ = -0.60 ($SiMe_3$), 20.29, 20.53 (OAc), 36.75 (C-2), 62.11 (C-6), 67.48, 68.55, 69.25 (C-3, C-4, C-5), 91.43

MS (70 eV): m/e = 347 (3 %, $M^+$-$CH_3$), 243 (7%), 229 (8%), 200 (16%), 185 (26%), 185 (26%), 161 (38%), 119 (76%), 117 (70%), 73 (47%, $Si(CH_3)_3$), 43 (100%, $CH_3CO$)

| | | | | | |
|---|---|---|---|---|---|
| $C_{15}H_{26}O_8Si$ (362.5) | Ber. | C | 49.71 | H | 7.23 |
| | Gef. | C | 49.88 | H | 7.21 |

Beispiel 2

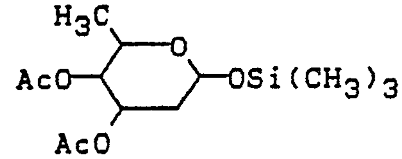

Trimethylsilyl-3,4-di-O-acetyl-2,6-didesoxy-α-L-arabino-hexopyranosid

4,20 g 3,4-Di-O-acetyl-2-desoxy-α,β-L-arabino-hexopyranose (18,1 mmol) wurden nach AAV1 umgesetzt. Nach einer halben Stunde wurde aufgearbeitet und im Vakuum destilliert. Sdp. 115 °C/0.03 Torr (Kugelrohr). Man erhielt 5.15 g (94 %) einer farblosen Flüssigkeit. Das Anomeren verhältnis betrug α:β = 80:20. $R_F$ = 0.72 (LS 1a).

$[\alpha]_D^{20}$ = -134.5° (c = 1, $CHCl_3$ α-Anomer).
IR (KBr): 2970 (C-H), 1740 (C=O), 1370 (C-H), 1250 (Si-O), 1230 (C-O), 1140, 1050 (C-O), 1010 (Si-O), 970, 890, 870, 850 (Si-C), 765 $cm^{-1}$ (Si-O)
$^1$H-NMR (200 MHz, $CDCl_3$): δ = 0,12 (s; 9 H, $Si(CH_3)_3$), 1,11 (d, $J_{6,5}$ = 6,5 Hz; 3 H, 6-$H_3$), 1,75 (ddd, $J_{2a,1}$ = 3,5 Hz, $J_{2a,2e}$ = 13.0 Hz, $J_{2a,3}$ = 11,5 Hz; 1 H, 2-$H_a$), 1.97, 2.02 (2xs; 6 H, OAc), 2.10 (ddd, $J_{2e,1}$ = 1,5 Hz, $J_{2e,2a}$, = 13.0 Hz, $J_{2,3}$ = 5,5 Hz; 1 H, 2-$H_e$), 3.95 (dq, $J_{5,6}$ = 6,5 Hz, $J_{5,6}$ = 10.0 Hz; 1H, 5-H), 4,70 (t, $J_{4,3}$ = $J_{4,5}$ = 9,5 Hz; 1H, 4-H), 5.25 (dd, $J_{1,2e}$ = 1,5 Hz, $J_{1,2a}$ = 3,5 Hz, 1H, 1-H), 5.32 (ddd, $J_{3,2e}$ = 5,5 Hz, $J_{3,2a}$ = 11,5 Hz, $J_{3,4}$ = 9,5 Hz; 1H, 3-H)
$^{13}$C-NMR (20 MHz, $CDCl_3$); δ = 0.60 ($Si(CH_3)_3$), 17.11 (C-6), 20.36, 20.54 (OAc), 37.08 (C-2), 65,16, 68.61 (C-3, C-5), 74.73 (C-4), 91,22 (C-1), 169,58, 169,67 (C=O). MS (70 eV): m/e = 289 (0,4 %, $M^+$-$CH_3$), 229 (2, M-$CH_3$-HOAc), 172 (10 %), 119 (16 %), 78 (8 %, $Si(CH_3)_3$), 43 (100 %, $CH_3CO$)

| | | | | | |
|---|---|---|---|---|---|
| $C_{13}H_{24}O_6Si$ (304,4) | Ber. | C | 51,29 | H | 7,95 |
| | Ber. | C | 51,46 | H | 7,99 |

Beispiel 3

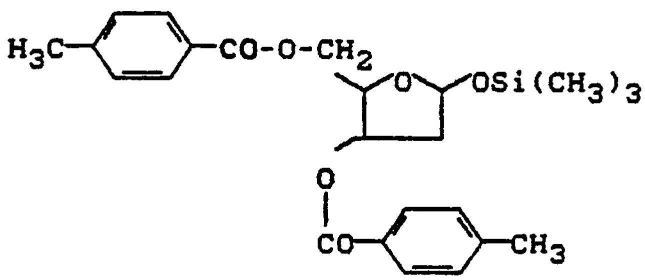

Trimethylsilyl-2-desoxy-3,5-di-O-(4-methylbenzoyl)-D-erythro-pentofuranosid

2-Desoxy-3,5-di-O-(4-methyl-benzoyl)-D-erythro-pentofuranosid (53.8 mmol) wurden nach der AAV1 umgesetzt. Aufarbeitung nach 0,5 h und Chromatographie an 140 g Kieselgel (60-200 µm) (LS 4c) ergaben 19.3 g (81 %) eines farblosen Öls. Diastereomerentrennung durch Chromatographie von 400 mg an 50 g Kieselgel (LS 4c) ergaben 169 mg des β-Anomeren.

$R_f$ = 0.37 (LS 4c) $[\alpha]_D^{20}$ = -3.2° (c = 0,5, $CHCl_3$) und 150 mg des α-Anomeren b $R_F$ = 0.27 (LS 4c) $[\alpha]_D^{20}$ = +81.4° (c = 0,5, $CHCl_3$)
IR (Film): 3040 (C-H), 2980 (C-H), 1720 (C=O), 1620 (C-C), 1450, 1410, 1380 (C-H), 1280 C-O), 1180, 1110, 1025, 850, 760, 700 $cm^{-1}$ (C=C)
$^1$H-NMR (200 MHz, $CDCl_3$): α-Anomeres: δ = 0.18 (s; 9H, $Si(CH_3)_3$), 2.19 (dd, $J_{2a,2e}$ = 14,5 Hz, $J_{2a,3}$ = 2,0 Hz; 1H, 2-$H_a$), 2,40, 2,42 (2xs; 6H $CH_3$), 2.49 (ddd, $J_{2e,1}$ = 5,0 Hz, $J_{2e,2a}$ = 14,5 Hz, $J_{2e,3}$ = 8,0 Hz; 1H, 2-$H_e$), 4.42-4.64 (m; 3 H, 4-H, 5-$H_2$) 5.46 (ddd, $J_{3,2e}$ = 8.0 Hz, $J_{3,2a}$ = 2,0 Hz, $J_{3,4}$ = 3.0 Hz; 1 H, 3-H), 5.72 (d, $J_{1,2a}$ = 5,0 Hz; 1 H, 1-H), 7.20-7.30 (m; 4H, Ph-H), 7.88-8.00 (m; 4 H, Ph-H) β-Anomeres: δ = 0,14 (s; 9 H, $Si(CH_3)_3$), 2,33 (dt, $J_{2a,1}$ = 5,0 Hz, $J_{2a,2e}$ = 14,0 Hz, $J_{2a,3}$ = 5,0 Hz; 1 H, 2-$H_a$), 2.40, 2.42 (2xs; 6 H, $CH_3$), 2.50 (ddd, $J_{2e,1}$ = 3,0 Hz, $J_{2e,2a}$ = 14,0 Hz, $J_{2e,3}$ = 7,0 Hz; 1 H, 2-$H_e$), 4.50 (s(br); 3 H, 4-H, 5-$H_2$), 5.60 (ddd, $J_{3,2e}$ = 7,0 Hz, $J_{3,2a}$ = 5,0 Hz, $J_{3,4}$ = 2,5 Hz; 1 H, 3-H), 5.72 (dd, $J_{1,2e}$ = 3.0 Hz, $J_{1,2a}$ = 5,0 Hz; 1 H, 1-H), 7.18-7.30 (m; 4 H Ph-H), 7.90-8.04 (m; 4 H, Ph-H)
$^{13}$C-NMR (20 MHz, $CDCl_3$): α-Anomeres: δ = -0.02 ($Si(CH_3)_3$), 21.41 ($CH_3$), 41.28 (C-2), 64.35 (C-6), 74,88, 81,35 (C-3, C-4), 98.79 (C-1), 126.98, 127,10, 128.88, 129,48, 129,56, 143.47, 143.60 (C-arom.), 165.98, 166,10 (C=O)
β-Anomeres: δ = -1.63 ($Si(CH_3)_3$), 19.87 ($CH_3$, 39.85 (C-2), 63.67 (C-6), 74.14, 79.88 (C-3, C-4), 97.61 (C-1), 125.25, 125.49, 127.28, 127.34, 127.96, 128.07, 141.84, 142.16 (C-arom.), 164.28, 164,52 (C=O) MS (70 eV): m/e = 427 (1%, $M^+$-$CH_3$), 306 (11%, M-$CH_3C_6H_4CO_2H$), 293 (11%), 216 (8%), 209 (9%), 193 (10%), 157 (22%), 119 (100%, $COC_6H_4CH_3$) 91 (19%, $CH_3C_6H_4$), 73 (21%, $Si(CH_3)_3$)

| | | | | | |
|---|---|---|---|---|---|
| $C_{24}H_{30}O_6Si$ (442.6) | Ber. | C | 65.13 | H | 6.83 |
| | Gef. | C | 65.24 | H | 6.99 |

Beispiel 4

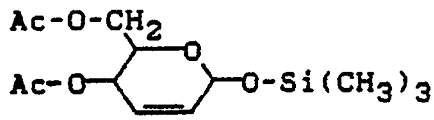

Trimethylsilyl 4,6-di-O-acetyl-2,3-di-desoxy-D-erythrohex-2-enopyranosid

5.00 g 3,4,6-Tri-O-acetyl-1,5-anhydro-2-desoxy-D-arabino-hex-1-enitol (18.4 mmol) wurden schnell unter Rühren in 100 ml kochendes Wasser gegeben und weiter unter Rückfluß erhitzt. Nach 14 min wurde die Lösung mit Hilfe eines Eisbades möglichst schnell auf Raumtemperatur gebracht. Man fügte langsam 1,8 g $BaCO_3$ hinzu. Danach extrahierte man 5 mal mit 30 ml Dichlormethan und trocknete die organische Phase mit $Na_2SO_4$. Die Lösung wurde in einen Photoreaktor aus Pyrex-Glas gegeben und bei -20° C mit einer 500 Watt Lampe 1 h lang bestrahlt. Entfernen des Lösungsmittels und Chromatographie an 2 mal 230 g Kieselgel (LS 1a) ergab 3.26 g eines farblosen Öls, das noch geringfügig durch dimere Nebenprodukte verunreinigt war. $R_F$ = 0.38 (LS 1a), $R_F$ = 0.40 (LS 1a, Dimer). Das Produkt wurde nach der AAV1 weiter umgesetzt. Nach 0,5 h wurde aufgearbeitet und im Vakuum destilliert. Sdp. 125° C/0,05 Torr. Man erhielt 3.31 g (60 %) eines farblosen Öls.

$R_F$ = 0,41 (LS 2e) $[\alpha]_D^{20}$ = +99.3° (c = 1, $CHCl_3$).

Diastereomerenverhältnis $\alpha : \beta$ = I : II = 4:1

IR (Film): 3040 (C=C), 2960, 2900 (C-H), 1740 (C=O), 1370 (C-H), 1250 (C-O), 1110, 1040 (Si-O), 980, 890, 850 $cm^{-1}$ (C=C)

$^1$H-NMR (200 MHz, $CDCl_3$): $\delta$ = 0,13 (S, 1.8 H, $Si(CH_3)_3$, II), 0.14 (s; 7.2 H, $Si(CH_3)_3$), I), 2.04, 2.06, (2xs; 6 H, OAc), 4.06-4.24 (m; 3 H, 6-$H_2$), 5-H), 5.10-5,42 (m, 2 H, 1-H, 4-H), 5.80 (s; 1,6 H, 2-H, 3-H, I), 5.84 (s; 0,4 H, 2-H, 3H, II),

$^{13}$-C-NMR (50.3 MHz, $CDCl_3$): $\delta$ = -0,3 ($Si(CH_3)_3$, I), 0.00 ($Si(CH_3)_3$, II), 20.38, 20.56 (OAc), 62.82, 66.52 (C-4, C-5, I), 88.61 (C-1, I), 90.98 (C-1, II), 125.66, 132.52 (C-2, C-3, II), 127.02, 129.82 (C-2, C-3, I), 169.74, 170.20 (C=O)

MS (70 eV): m/e = 287 (0.6 % $M^+$-$CH_3$), (8%, M-$CH_3CO_2$), 229 (1%, M-Aglycon), 200 (34%, AcO(CH)-$_4$OSi$(CH_3)_3$), 158 (100 %, HO$(CH)_4$OSi$(CH_3)_3$, 117 (65%), 73 (66%, $Si(CH_3)_3$), 43 (100 %, $CH_3CO$)

| | | | | | |
|---|---|---|---|---|---|
| $C_{13}H_{22}O_6Si$ (302.4) | Ber. | C | 51.63 | H | 7.33 |
| | Gef. | C | 51.78 | H | 7.46 |

Beispiel 5

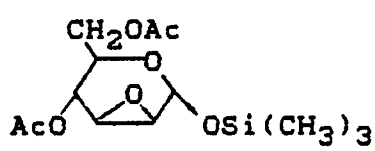

Trimethylsilyl-4,6-di-O-acetyl-2,3-anhydro-$\alpha$-D-mannopyranosid

2,18 g 4,6-Di-O-acetyl-2,3-anhydro-α-D-mannopyranose (8.85 mmol) wurden nach der AAV1 umgesetzt. Nach 0.5h wurde aufgearbeitet und das restliche Pyridin über Nacht bei Raumtemperatur in Ölpumpenvakuum entfernt. Kristallisation aus Pentan ergaben 2.46 g (89 %) Produkt. Smp.

60° C, $R_F$ = 0,45 (LS 1a) $[\alpha]_D^{20}$ = +62.5° (c = 1 $CHCl_3$)

IR (Film): 3020, 2970, 2920 (C-H), 1750 (C=O), 1370 (C-H), 1260, 1230 (C-O), 1140, 1070, 1040, 890, 850 $cm^{-1}$

$^1$H-NMR (200 MHz, $CDCl_3$): δ = 0.18 (s; 9 H, $Si(CH_3)_3$, 2.24, 2.31 (2xs; 6 H, OAc), 3.01 (dd, $J_{3,2}$ = 3,5 Hz, $J_{3,4}$ = 1,0 Hz; 1 H, 3-H), 3.20 (d, $J_{2,3}$ = 4,5 Hz, 1H 2-H), 3.88-3.98 (mc; 1 H, 5-H), 4.04-4.10 (m; 2 H, 6-$H_2$), 4.84 (dd, $J_{4,3}$ = 1.0 Hz, $J_{4,5}$ = 10.0 Hz; 1 H, 4-H), 5.32 (s; 1 H, 1-H)

$^{13}$C-NMR (50 MHz, $CDCl_3$): δ = -0.26 ($Si(CH_3)_3$), 20.58, 20.67 (OAc), 50.45, 52.96 (C-2, C-3), 62.85, 64.26 (C-4, C-5), 63.00 (C-6), 89.50 (C-1), 169.28, 170.38 (C=O)

MS (70 eV): m/e = 303 (10 %, $M^+$-$CH_3$), 258 (2%, M-HOAc), 216 (10%, M-HOAc-Keten), 174 (33 %, $HOCCHCHOCH_2OSiMe_3$), 117 (65 %), 98 (91 %), 74 (66 %, $Si(CH_3)_3$), 43 (100 %, $CH_3CO$)

| | | | | | |
|---|---|---|---|---|---|
| $C_{13}H_{22}O_7Si$ (318.4) | Ber. | C | 49.05 | H | 6.96 |
| | Gef. | C | 49.31 | H | 7.13 |

Beispiel 6

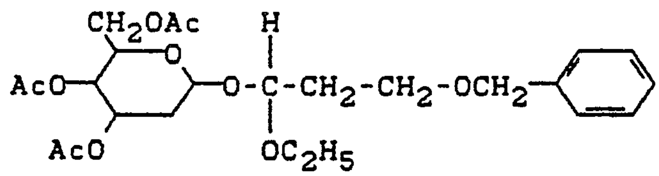

(1′RS)-1′-Ethoxy-3′-phenylmethoxy-propyl-3,4,6-tri-O-acetyl-2-desoxy-α-D-arabino-hexopyranosid

500 mg von Beispiel 1 (1.38 mmol) wurden nach der AAV2 mit 3-Phenylmethoxypropionaldehyddiethylacetal (A) und 3-Phenylmethoxypropionaldehyd bei -70° umgesetzt. Nach 24 h werden noch einmal die halbe Menge Trifluormethansulfonsäuretrimethylsilylester, A und 3-Phenylmethoxypropionaldehyd hinzugegeben.

Nach 48 h wurde abgebrochen, aufgearbeitet und an 80 g Kieselgel (LS 2b) chromatographiert. Man erhielt 579 mg (87 %) eines farblosen Öls. $R_F$ = 0.17 (LS 3a)

$[\alpha]_D^{20}$ = +84.7° (c = 1, $CHCl_3$. Diastereomerenverhältnis:

(1R, 1′R) : (1R, 1′S) = I:II = 80:20

IR (Film): 2990 (C-H), 1740 (C=O), 1370 (C-H), 1230 (C-O), 1140, 1100, 1040, 980, 740, 700 $cm^{-1}$

$^1$H-NMR (200 MHz, $CDCl_3$): δ 1.16 (t, $J_{2'',1''}$ = 7.0 Hz; 0,75 H, 1″-H, II), 1.18 (t, $J_{2'',1''}$ = 7,0 Hz; 2,25 H, 1″-H, I), 1.87 (ddd, $J_{2a,1}$ = 4.0 Hz, $J_{2a,2e}$ = 13.0 Hz, $J_{2a,3}$ = 11.5 Hz; 1 H, 2-$H_a$) 1.92-2.10 (m; 2 H, 2′-$H_2$), 2.02, 2.04 (2xs; 9 H, OAc), 2,36 (ddd, $J_{2e,1}$ = 1,5 Hz, $J_{2e,2a}$ = 13.0 Hz, $J_{2e,3}$ = 5,5 Hz; 1 H , 2-$H_e$), 3.44-3.86 (m; 4 H, 3′-$H_2$, 1″-$H_2$), 3.92-4.16 (m; 2 H, 6-H, 5-H), 4.24 (dd, $J_{6,6'}$ = 12,5 Hz, $J_{6,5}$ = 5.0 Hz; 0,75 H, 6′-H, I), 4.24-4.34 (m; 0,25 H, 6′-H, II), 4.50 (s(br); 2 H, $CH_2$-Phenyl), 4.84 (t, $J_{1',2'}$ = 6.0 Hz; 0,25 H, 1′-H, II) 4.92 (t, $J_{1',2'}$ = 6.0 Hz; 0,75 H, 1′-H, I), 5.00 (t, $J_{4,3}$ = $J_{4,5}$ = 9,5 Hz; 0.75 H, 4-H, I), 5.01 (t, $J_{4,3}$ = $J_{4,5}$ = 9,5 Hz; 0,25 H, 4-H, II), 5.22 (dd, $J_{1,2e}$ = 1,5 Hz, $J_{1,2a}$ = 4,0 Hz; 0,25 H, 1-H, II), 5.31 (dd, $J_{1,2e}$ = 1,5 Hz, $J_{1,2a}$ = 4,0 Hz; 0,75 H, 1-H, I), 5,34 (ddd, $J_{3,2e}$ = 5.5 Hz; $J_{3,2a}$ = 11,5 Hz, $J_{3,4}$ = 9,5 Hz; 1H, 3-H), 7.36 (s(br); 5 H, Phenyl-H)

$^{13}$C-NMR (50.3 MHz, $CDCl_3$): δ = 15.20 (C-2″, II), 15.33 (C-2″, I), 20.65, 20.71, 20.96 (OAc), 34.70, 34.83 (C-2, C-2′, I), 35,27, 35.51 (c-2, C-2′, II), 62.27 (C-1″, I), 62.45 (C-6), 63.74 (C-1″, II), 65.96 (C-3′, II), 66.13 (C-3′, I), 68.28. 69.03, 69.33 (C-3, C-4, C-5, I), 68.42, 69.20 (C-3, C-4, C-5, II), 73.00 (Phenyl-$CH_2$-O), 92,98 (C-1, I) 93.80 (C-1, II) 97.86 (C-1′, I), 101.77 (C-1′, II), 127.54, 127.60, 127.73, 128.35 (C-2, C-3, C-5, arom.), 138.25 (C-1, arom.), 169.82, 170.11 170.19 (C=O)

MS (70 eV): m/e = 274 (1%, M + $H^+$-Aglycon), 273 (8%, M-Aglycon), 214 (4%, M+H-Aglycon-HOAc), 213 (33 %, M-Aglycon-HOAc), 193 (21 %, $CH(OC_2H_5)(CH_2)_2$-O-$CH_2$-Phenyl), 153 (26 %, M-Aglycon-2xHOAc),

111 (M-Aglycon-2 x HOAc-Keten), 91 (96 %, $CH_2$-$C_6H_5$), 87 (51 %), 86 (96 %), 43 (100 %, $CH_3CO$).

| | | | | | |
|---|---|---|---|---|---|
| $C_{24}H_{34}O_{10}$ (482.5) | Ber. | C | 59,74 | H | 7.10 |
| | Gef. | C | 59,90 | H | 7,22 |

Beispiel 7

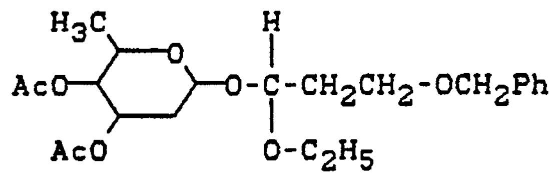

(1'RS)-1'-Ethoxy-3'-phenylmethoxy-propyl-3,4-di-O-acetyl-2,6-di-desoxy-α-L-arabino-hexopyranosid

400 mg Trimethylsilyl 3,4-Di-O-acetyl-2,6-didesoxy-α-L-arabino-hexopyranosid (Beispiel 2) (1.31 mmol) wurde nach der AAV2 mit 3-Phenylmethoxypropionaldehyddiethylacetal und 3-Phenylmethoxypropanal bei -78° C umgesetzt. Nach 24 h gab man die halbe Menge an Trifluormethansulfonsäuretrimethylsilylester, 3-Phenylmethoxypropionaldehyddiethylacetal und 3-Phenylmethoxypropanal hinzu. Nach 48 h wurde aufgearbeitet und an 80 g Kieselgel (LS 2c) chromatographiert. Man erhielt 517 mg (93 %) eines farblosen Öls.

$R_F$ = 0.33 (LS 2e). $[\alpha]_D^{20}$ = -112.0° (c = 1, $CHCl_3$). Diastereomerenverhältnis (1S, 1'S) : (1S, 1'R) = I : II 75:25

IR (Film): 3040 (C-H), 2980, 2940 (C-H), 2880 (C-H), 1750 (C=O), 1370 (C-H), 1250, 1240 (C-O), 1140, 1050, 980, 740 + 705 $cm^{-1}$ (C-C, arom. monosubst.)

$^1$H-NMR (200 MHz, $CDCl_3$): δ = 1.10 (d, $J_{6,5}$ = 6.0 Hz; 0.75 H, 6-$H_3$, I), 1.12 (d, $J_{6,5}$ = 6,0 Hz; 0,25 H, 6-$H_3$, II), 1.17 (t, $J_{2'',1''}$ = 7,0 Hz; 0,75 H, 2''-Hg II), 1.18 (t, $J_{2'',1''}$ = 7,0 Hz; 2,25 H, 2''-$H_3$, I), 1.82 (ddd, $J_{2a,1}$ = 4,0 Hz $J_{2a,2e}$ = 13.0 Hz, $J_{2a,3}$ = 11,5 Hz; 1 H, 2-$H_a$), 1.90-2.03 (m; 2 H, 2'-$H_2$), 2.01, 2.04 (2xs; 6H, OAc), 2.23 (ddd, $J_{2e,1}$ = 1,5 Hz, $J_{2e,2a}$ = 13.0 Hz, $J_{2e,3}$ = 5,5 Hz; 1 H, 2-$H_e$), 3.40-3.77 (m; 2H, 1''-$H_2$), 3.59 (t, $J_{3',2'}$ = 6,0 Hz; 2 H, 3'-$H_2$) 3.86 (dq, $J_{5,6}$ = 6,5 Hz, $J_{5,4}$ = 9,5 Hz; 0,75 H, 5-H, I) 4.01 (dq, $J_{5,6}$ = 6,5 Hz, $J_{5,4}$ = 9,5 Hz; 0.25 H, 5-H, II), 4,41-4,57 (m; 2 H, $CH_2$-$C_6H_5$), 4.73 (t, $J_{4,5}$ = $J_{4,3}$ = 9,5 Hz; 1 H, 4-H), 4.80 (t, $J_{1',2'}$ = 5,5 Hz; 0,25 H, 1'-H, II), 4.91 (t, $J_{1',2'}$ = 6,0 Hz; 0,75 H, 1'-H, I), 5.09 (dd, $J_{1,2e}$ = 1,5 Hz, $J_{1,2a}$ = 4,0 Hz; 0,25 H, 1-H, II), 5.21 (dd, $J_{1,2e}$ = 1,5 Hz, $J_{1,2a}$ = 4,0 Hz, 0.75 H, 1-H, I), 5.28 (ddd, $J_{3,2e}$ = 5,5 Hz, $J_{3,2a}$ = 11,5 Hz, $J_{3,4}$ = 9.5 Hz; 1 H, 3-H), 7,20-7,40 (m, 5H, Phenyl-H).

$^{13}$C-NMR (50.3 MHz, $CDCl_3$): δ = 15.18 (C-2'', II), 15.33 (C-2'', I), 17.51 (C-6), 20.74, 20.93 (OAc), 34,68, 35,10 (C-2, C-2', I), 35.29, 35,74 (C-2, C-2', II), 62.08 (C-1'', I), 63.42 (C-1'', II), 65.95 (C-3', II), 66,17 (C-3', I); 66.02, 68.95 (C-3, C-5, I), 66.08, 69.01 (C-3, C-5, II), 72,87 (O-$CH_2$-$C_6H_5$), I), 72.92 (O-$CH_2$-$C_6H_5$, II), 74,72 (C-4), 92.72 (C-1, I), 93.76 (C-1, II), 97.44 (C-1', I), 101.51 (C-1', II) 127.37, 127.45, 127.59, 128.18 (C-2, C-3, C-4, arom.), 136-17, 136.23 (C-1, arom.), 169.83, 169.90 (C=O)

MS (200 eV, DCI, $NH_3$): m/e = 442 (100 %, M + $NH_4^+$)

| | | | | | |
|---|---|---|---|---|---|
| $C_{22}H_{32}O_8$ (424,5) | Ber. | C | 62.25 | H | 7.60 |
| | Gef. | C | 62.33 | H | 7.55 |

Beispiel 8

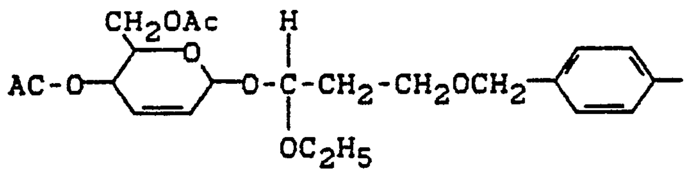

(1′RS)-1′-Ethoxy-3′-phenyl-methoxy-propyl-4,6-di-O-acetyl-2,3-di-desoxy-D-erythro-hex-2-enopyranosid

300 mg der Verbindung aus Beispiel 4 (1.02 mmol) wurden nach der AAV2 mit Phenylmethoxypropionaldehyddiethylacetal und 3-Phenylmethoxypropanal bei -90° C umgesetzt. Nach 24 h wurde aufgearbeitet und an 70 g Kieselgel (LS 6) chromatographiert. Man erhielt 281 g (65 %) eines farblosen Öls. $R_F$ = 0,53 (LS 1a) $[\alpha]_D^{20}$ = +94,7° (c = 1, $CHCl_3$). Das Produktgemisch bestand aus 2 α-konfigurierten Diastereomeren im Verhältnis (1R, 1′R) : (1R, 1′S) = I:II = 66:34.

IR (Film): 3050 (C-H), 3020 (C=C), 2980, 2930 (C-H), 2870 (C-H), 1740 (C=O) 1370 (C-H), 1240 (C-O), 1100, 1030, 970, 740, 700 $cm^{-1}$

$^1$H-NMR (200 MHz, $CDCl_3$): δ = 1.19 (t, $J_{2'',1''}$ = 7.0 Hz; 3 H, 2″-$H_3$), 190-2.08 (m; 2 H, 2′-$H_2$, 2.04, 2.09 (2xs; 6 H, OAc), 3.42-3.56 (m; 1 H, 1″-H), 3.56 (t, $J_{3',2'}$ = 6,0 Hz; 1.32 H, 3′-$H_2$, I), 3.58 (t, $J_{3',2'}$ = 6,0 Hz; 0,66 H, 3′-$H_2$, II), 3.70 (dq, $J_{1''',2''}$ = 7,0 Hz, $J_{1''',1''}$ = 9,5 Hz; 0.66 H, 1‴-H, I) 3.82 (dq, $J_{1''',1''}$ = 9,5 Hz; 0.33 H, 1‴-H, II), 4.02-4.32 (m; 3 H, 6-$H_2$, 5-H), 4.48 (A-Teil vom AB-System, J = 12.0 Hz; 1 H, $CH_2$-$C_6H_5$), 4.51 (B-Teil vom AB-System, J = 12,0 Hz; 1 H, $CH_2$-$C_6H_5$), 4.91 (t, $J_{1',2'}$ = 6,0 Hz; 0,33 H, 1′H, II), 5.00 (t, $J_{1',2'}$ = 5,5 Hz; 0.66 H, 1′-H, I), 5.24-5.38 (m; 2 H, 1-H, 4-H), 5.66-6.00 (m; 2 H, 2-H, 3-H), 7.34 (s(br); 5 H, $C_6H_5$)

$^{13}$C-NMR (20 MHz, $CDCl_3$): δ = 15.16 (C-2″, II), 15.32 (C-2″, I), 20.50, 20.59, 20.78 (OAc), 35.22 (C-2′, I), 35.52 (C-2′, II), 62.31, 62.98, 63.13, 63.38 (C-1″, C-6), 65.07, 67.41 (C-4, C-5, II), 65.26, 67.25 (C-4, C-5, I), 66.02 (C-3′, II), 66.15 (C-3′, I) 72.92 ($OCH_2$-$C_6H_5$), 90.40 (C-1, II), 91.14 (C-1, I), 99.39 (C-1′, I), 101.32 (C-1′, II), 127.50, 127.59, 127.64, 127.79, 128.14, 128.29, 128.71, 129.12 (C-2, C-3, C-4 arom., C-2, C-3), 138.44 (C-1 arom.), 169,92, 170.32, 170.37 (C=O)

MS (70 eV): m/e = 213 (21 %, $M^+$-Aglycon), 163 (31 %), 153 (17 %, M-Aglycon-HOAc), 111 (39 % M-Aglycon-HOAc-Keten), 91 (83 %, $CH_2$-$C_6H_5$), 86 (52 %, $C_2H_5O$-CH-$CH_2$-$CH_2$), 43 (100 %, $CH_3CO$)

| | | | | | |
|---|---|---|---|---|---|
| $C_{22}H_{30}O_8$ (422.5) | Ber. | C | 62.55 | H | 7.15 |
| | Gef. | C | 62.42 | H | 7.14 |

Beispiel 9

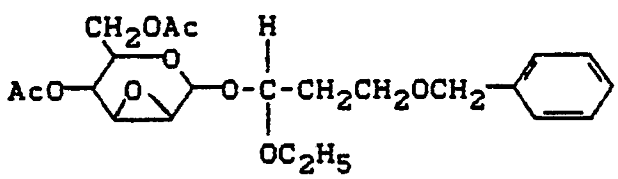

(1′RS)-1′-Ethoxy-3′-phenylmethoxy-propyl-4,6-di-O-acetyl-2,3-anhydro-α-D-mannopyranosid

141 mg der Verbindung aus Beispiel 5 (0.44 mmol) wurden nach der AAV2 mit 3-Phenylmethoxypropionaldehyddiethylacetal und 3-Phenylmethoxypropionaldehyd bei -70° C umgesetzt. Nach 24 h wurde aufgearbeitet und an 20 g Kieselgel (LS 2b) chromatographiert. Man erhielt 100 mg (51 %) eines farblosen Öls. $R_F$ = 0.29 (LS 2e) $[\alpha]_D^{20}$ = +67.8° (c = 1, $CHCl_3$). Diastereomerenverhältnis (1R, 1′R): (1R, 1′S) = I:II = 60:40,

IR (Film): 2980, 2940 (C-H), 2870 (C-H), 1750 (C=O), 1370 (C-H), 1240 (C-O), 1140, 1100, 1040, 970, 740, 700 $cm^{-1}$ (C=C)

$^1$H-NMR (200 MHz, $CDCl_3$): 1 Diastereomer: $\delta$ = 1.21 (t, $J_{2'',1''}$ = 7,0 Hz; 3 H, 2''-$H_3$), 1.93-2.09 (m; 2 H, 2'-$H_2$), 2.00, 2.12 (2xs; 6 H, OAc), 3.13 (dd, $J_{3,2}$ = 3,5 Hz, $J_{3,4}$ = 1,0 Hz; 1 H, 3-H), 3.24 (d, $J_{2,3}$ = 3,5 Hz; 1 H, 2-H), 3.47-3.64 (m: 3 H, 1''-H, 3'-$H_2$), 3.73 (dq, $J_{1''',2''}$ = 7,0 Hz, $J_{1''',1''}$ = 9,5 Hz; 1 H, 1'''-H), 3.83-3.94 (mc; 1 H, 5-H), 4.03-4.14 (m; 2 H, 6-$H_2$), 4.47 (A-Teil vom AB-System, J = 12.0 Hz; 1 H, $CH_2$-Ph), 4.51 (B-Teil vom AB-System, J = 12.0 Hz; 1 H, $CH_2$-Ph), 4.85 (dd, $J_{4,3}$ = 1,0 Hz, $J_{4,5}$ = 10.0 Hz; 1 H, 4-H), 5.01 (t, $J_{1',2'}$ = 5,5 Hz: 1 H, 1'-H), 5.33 (s; 1 H, 1-H), 7.24-7.40 (m; 5 H, Ph-H)

$^{13}$C-NMR (50 MHz, $CDCl_3$): 1 Diastereomer: $\delta$ = 15.28 (C-2''), 20.57, 20.76 (OAc), 35.72 (C-2'), 49.20, 53.18 (C-2, C-3), 62.90, 63.14 (C-6, C-1''), 62.97, 65.04 (C-4, C-5), 65,91 (C-3'), 73.00 ($CH_2$-Ph), 91.47 (C-1), 99.20 (C-1'), 127.48, 127.53, 128.22 (C-2, C-3, C-4, arom). 138.06 (C-1, arom.), 169.33, 170.47 (C=O)

MS (200 eV, DCI, $NH_3$): m/e = 456 (100 %, M + $NH_4^+$)

| | | | | | |
|---|---|---|---|---|---|
| $C_{22}H_{30}O_9$ (438.5) | Ber. | C | 60.26 | H | 6.90 |
| | Gef. | C | 60.17 | H | 7.00 |

Beispiel 10

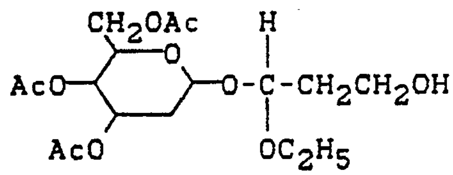

(1'RS)-1'-Ethoxy-3'-hydroxy-propyl-3,4,6-tri-O-acetyl-2-desoxy-α-D-arabino-hexopyranosid

500 mg der Verbindung aus Beispiel 1 (1.04 mmol) wurden nach der AAV4 umgesetzt. Die Reaktionsdauer betrug 0,5 h. Säulenchromatographie nach Aufarbeitung an 30 g Kieselgel (LS 1c) erbrachten 393 mg (96 %) eines farblosen Öls. $R_F$ = 0.10 (LS 1a).

$[\alpha]_D^{20}$ = +102° (c = 1, $CHCl_3$). (1R, 1'R) = I; (1R, 1'S) = II

IR (Film): 3500 (O-H), 2990 (C-H), 1740 (C=O), 1370 (C-H), 1230 (C-O), 1130, 1040, 970 $cm^{-1}$

$^1$H-NMR (200 MHz, $CDCl_3$): $\delta$ 1,21 (t, $J_{2'',1'''}$ = 7,0 Hz; 0.75 H, 2''-$H_3$, II), 1.43 (t, $J_{2'',1''}$ = $J_{2'',1'''}$ = 7,0 Hz; 2.25 H, 2''-$H_3$, I), 1.82-2.12 (m; 4 H, 2-$H_a$, OH), 2.03, 2.05, 2.09 (3xs; 9 H, OAc), 2.21 (ddd, $J_{2e,1}$ = 1,5 Hz, $J_{2e,2a}$ = 13.0 Hz, $J_{2e,3}$ = 5,5 Hz; 0,25 H, 2-$H_e$, II), 2.30 (ddd, $J_{2e,1}$ = 1,5 Hz, $J_{2e,2a}$ = 13.0 Hz, $J_{2e,3}$ = 5.5 Hz; 0,75 H, 2-$H_e$, I), 3.43-3.94 (m; 4 H, 1'''-H, 3'-$H_2$), 3.98-4.14 (m; 1.75 H, 6-H, I+II, 5-H, I), 4.13-4.23 (m; 0.25 H, 5-H, II), 4.28 (dd, $J_{6',5}$ = 5,5 Hz, $J_{6',6}$ = 12.5 Hz; 0.75 H, 6'-H, I), 4.32 (dd, $J_{6',5}$ = 4,5 Hz, $J_{6',6}$ = 12.0 Hz; 0,25 H, 6'-H, II), 3.86 (dd, $J_{1',2'}$ = 5,0 Hz, $J_{1',2}$ = 7.0 Hz; 0,25 H, 1'-H, II), 4.93 (t, $J_{1',2'}$ = 5,0 Hz, $J_{1',2'}$ = 7.0 H; 0,25 H, 1'-H, II), 4.93 (t, $J_{1',2'}$ = 5,5 Hz; 0.75 H, 1'-H, I), 5.00 (t, $J_{4,3}$ = $J_{4,5}$ = 10.0 Hz; 0,75 H, 4-H, I), 5.04 (t, $J_{4,3}$ = $J_{4,5}$ = 10.0 Hz; 0.25 H; 4-H, II), 5.29-5.33 (m; 1 H, 1-H), 5.34 (ddd, $J_{3,2e}$ = 5,5 Hz, $J_{3,2a}$ = 11,5 Hz, $J_{3,4}$ = 10.0 Hz; 0.75 H, 3-H, I), 5.36 (ddd, $J_{3,2e}$ = 5,5 Hz, $J_{3,2a}$ = 11,5 Hz, $J_{3,4}$ = 10.0 Hz; 0,25 H, 3-H, II)

$^{13}$C-NMR (50.3 MHz, $CDCl_3$): $\delta$ = 14.68 (C-1'', II), 14.76 (C-2'', I), 20.1, 20.33 (OAc), 34.31, 36.50 (C-2, C-2', I), 35.10, 36,84 (C-2, C-2', II), 57,77 (C-3', I), 57.88 (C-3', II), 61.81, 61.98 (C-6), C-1'', I), 63.19 (C-1'', II), 67.85, 68.03, 68.45, 68.97 (C-3, C-4, C-5), 93.00 (C-1), 98.50 (C-1', I), 101.54 (C-1', II), 169.37, 169,59, 170.16 (C=O).

MS (70 eV): m/e = 273 (6 %, $M^+$-Aglycon), 214 (3 %, M + H-Aglycon-HOAc), 213 (29 %, M-Aglycon-HOAc), 153 (22 %, M-Aglycon-2xHOAc), 111 (22 %, M-Aglycon-2xHOAc-Keten), 103 (95 %, $CH(OC_2H_5)$-$CH_2CH_2OH$), 59 (53 %), 45 (52 %), 43 (100 %, $CH_3CO$)

| | | | | | |
|---|---|---|---|---|---|
| $C_{17}H_{28}O_{10}$ (392.4) | Ber. | C | 52,04 | H | 7,19 |
| | Gef. | C | 51,87 | H | 7,21 |

Beispiel 11

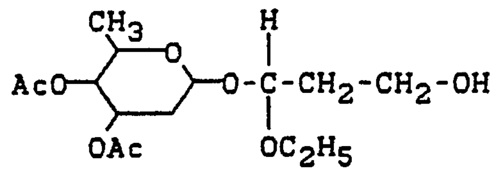

(1′RS)-1′-Ethoxy-3′-hydroxy-propyl-3,4-di-O-acetyl-2,6-didesoxy-α-L-arabino-hexopyranosid

800 mg der Verbindung aus Beispiel 7 (1.89 mmol) wurden nach der AAV4 für 20 min umgesetzt. Anschließende Chromatographie an 20 g Kieselgel (LS 1a) ergaben 613 mg (97 %) eines farblosen Öls. $R_F$ = 0.37 (LS 1a) $[\alpha]_D^{20}$ = -121.3° (c = 1, $CHCl_3$), (1S, 1′S) = I, (1S, 1′R) = II

IR (Film): 3500 (O-H), 2990, 2940 (C-H), 2880 (C-H), 1740 (C=O), 1370 (C-H), 1250, 1230 (C-O), 1140, 1050, 980 $cm^{-1}$

$^1$H-NMR (200 MHz, $CDCl_3$): δ = 1.17 (d, $J_{6,5}$ = 6.0 Hz; 3 H, 6-$H_3$), 1.22 (t, $J_{2'',1''}$ = 7.0 Hz; 3 H, 2″-$H_3$), 1.84 (ddd, $J_{2a,1}$ = 4.0 Hz, $J_{2a,2e}$ = 13.0 Hz, $J_{2a,e}$ = 11.5 Hz; 1 H, 2-$H_a$), 1.86-2.08 (m; 2H, 2′-$H_2$), 2.01, 2.05 (2sx; 6 H, OAc), 2.19 (ddd, $J_{2e,1}$ = 1,5 Hz, $J_{2e,2a}$ = 13.0 Hz, $J_{2e,3}$ = 5,5 Hz; 0.25 H, 2-$H_e$, II), 2.27 (ddd, $J_{2e,1}$ = 1,5 Hz, $J_{2e,2a}$ = 13.0 Hz, $J_{2e,3}$ = 5,5 Hz; 0,75 H, 2-$H_e$, I), 3.51 (dq, $J_{1'',2''}$ = 7.0 Hz, $J_{1'',1'''}$ = 9,5 Hz, 1 H, 1″-H), 370 (dq, $J_{1''',2''}$ = 7.0 Hz, $J_{1''',1''}$ = 9,5 Hz; 1 H, 1‴-H), 3.68-3.87 (m; 2 H, 3′-$H_2$), 3.87 (dq, $J_{5,6}$ = 6,0 Hz, $J_{5,4}$ = 9,5 Hz; 0,75 H, 5-H, I), 4.03 (dq, $J_{5,6}$ = 6,0 Hz, $J_{5,4}$ = 9,5 Hz; 0.25 H, 5-H, II), 4,7 (t, $J_{4,5}$ = $J_{4,3}$ = 9,5 Hz; 0,75 H, 4-H, I), 4.76 (t, $J_{4,5}$ = $J_{4,3}$ = 9,5 Hz; 0,25 H, 4-H, II), 4.89 (t, $J_{1',2'}$ = 5,5 Hz, 1 H, 1′-H), 5.16 (dd, $J_{1,2e}$ = 1,5 Hz, $J_{1,2a}$ = 4.0 Hz; 0.25 H, 1-H, II), 5.20 (dd, $J_{1,2e}$ = 1,5 Hz, $J_{1,2a}$ = 4,0 Hz; 0.75 H, 1-H, I), 5,25 (ddd, $J_{3,2e}$ = 5,5 Hz, $J_{3,2a}$ = 11.5 Hz, $J_{3,4}$ = 9,5 Hz; 1 H, 3-H)

$^{13}$C-NMR (50.3 MHz, $CDCl_3$): δ = 15.21 (C-2″, II), 15.27 (C-2″, I), 17.51 (C-6, I), 17.55 (C-6, II), 20.80, 20.95 (OAc), 35.05, 35.98 (C-2,C-2′, I), 35.73, 37.11 (C-2, C-2′, II), 58.54 (C-3′, I), 58.63 (C-3′, II), 62.47 (C-1″, I), 63,60 (C-1″, II), 66.16, 68.89 (C-3,C-5, I), 66.28, 69.03 (C-3, C-5, II), 74.60 (C-4), 93.49 (C-1, II), 93.61 (C-1, I), 99.40 (C-1′, I), 102.05 (C-1′, II), 170.06, 170.13 (C=O)

MS (200 eV, DCI, $NH_3$): m/e = 352 (100 %, M + $NH_4^+$)

| | | | | | |
|---|---|---|---|---|---|
| $C_{15}H_{26}O_8$ (334.4) | Ber. | C | 53.88 | H | 7.84 |
| | Gef. | C | 53.83 | H | 7.93 |

Beispiel 12

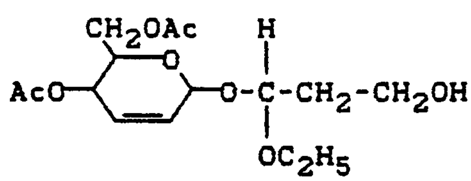

(1′RS)-1′-Ethoxy-3′-hydroxy-4,6-di-O-acetyl-2,3-di-desoxy-D-erythro-hex-2-enopyranosid

680 mg (1′RS)-1′-Ethoxy-3′-(4-methoxy-phenyl)-methoxy-propyl-4,6-di-O-acetyl-2,3-di-desoxy-D-erythro-hex-2- enopyranosid (1,51 mmol) werden in einer Suspension aus 90 ml Dichlormethan und 5 ml Wasser gelöst. Man gibt 451 mg 2,3-Dichlor-5,6-dicyan-p-benzochinon (1.99 mmol) bei Raumtemperatur hinzu. Das Reaktionsgemisch färbt sich grün und ändert während der 4h 15 min Reaktionszeit seine Farbe nach gelb. Die anfänglich klare Emulsion wird durch ausfallendes Hydrochinon trübe. Nach Beendigung der Reaktion (DC-Kontrolle) wird mit 50 ml eines Puffers (pH 7) versetzt. Nach Abtrennen des Dichlormethans

wird die schwarze wäßrige Phase zweimal mit Dichlormethan extrahiert. Nach Trocknung und Entfernung des Lösungsmittels wird möglichst schnell säulenchromatographisch an 22 g Kieselgel (LS 1c) gereinigt, da sonst das entstandene Hydrochinon das Glycosid spaltet. Man erhält 422 g (84 %) eines farblosen Öls. $[\alpha]_D^{20}$ = +115.8 (c = 0,5, $CHCl_3$). Diasteromerenverhältnis: (1R, 1′R) : (1R, 1′S) = I : II = 60 : 40.

IR (Film): 3500 (O-H), 2980, 2930, 2890 (C-H), 1740 (C=O), 1370 (C-H), 1240 (C-O), 1130, 1000, 960 $cm^1$

$^1$H-NMR (200 MHz, $CDCl_3$); δ = 1.24 (t, $J_{2'',1''}$ = 7.0 Hz; 1.8 H; 2″-$H_3$, I), 1.25 (t, $J_{2'',1''}$ = 7,0 Hz, 1.2 H, 2″-$H_3$, II), 1.97 (q(br), $J_{2',1'}$ = $J_{2',3'}$ = 6,0 Hz; 2 H, 2′-$H_2$), 1,90-2.05 (m; 1 H, OH, $D_2O$-Austausch), 2.10 (s(br); 6 H, OAc), 3.46-3.99 (m; 4 H, 3′-$H_2$, 1″-$H_2$), 4.02-4.28 (m; 3 H, 5-H, 6-$H_2$), 4.92 (t, $J_{1',2'}$ = 5,5 Hz; 0,4 H, 1′-H, II), 5.02 (t, $J_{1',2'}$ = 5,5 Hz; 0,6 H, 1′-H, I), 5.24-5.48 (m; 2 H, 1-H, 4-H), 5.73-5.98 (m; 2 H, 2-H, 3-H)

$^{13}$C-NMR (50 MHz, $CDCl_3$): δ = 15.19 (C-2″, II), 15.30 (C-2″, I), 20.73, 20.78, 20.97 (OAc), 36.99 (C-2′, II), 37.16 (C-2′, I), 58.73 (C-1″, I), 58.85 (C-1″, II), 62.79, 63.11, 63.16, 63.80 (C-6, C-3′), 65.01, 67.48 (C-4, C-5, II), 65.22, 67.32 (C-4, C-5, I), 90.30, (C-1, II), 91.91 (C-1, I) 101.25 (C-1′, I), 102.34 (C-1′, II), 127.25, 129.46 (C-2, C-3, I), 127.71, 129.14 (C-2, C-3, II), 170.25, (170,74 (C=O) MS (200 eV, DCI, $NH_3$): m/e = 350 (100 %, M + $NH_4^+$)

| | | | | | |
|---|---|---|---|---|---|
| $C_{15}H_{24}O_8$ (332.4) | Ber. | C | 54.21 | H | 7.28 |
| | Gef. | C | 53.81 | H | 7.57 |

Beispiel 13

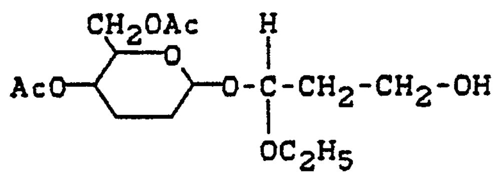

(1′RS)-1′-Ethoxy-3′-hydroxy-propyl-4,6-di-O-acetyl-2,3-di-desoxy-α-D-erythro-hexopyranosid

300 mg (1′RS)-1′-Ethoxy-3′-phenyl-methoxy-propyl-4,6-di-O-acetyl -2,3-didesoxy-D-erythro-hex-2-eno-pyranosid (Beispiel 8) (0,71 mmol) wurden nach der AAV4 umgesetzt. Nach 30 min arbeitete man auf und chromatographierte an 20 g Kieselgel (LS 1d). Man erhielt 196 mg (83 %) eines farblosen Öls. $R_F$ = 0.16 (LS 1a) $[\alpha]_D^{20}$ = +116.4° (c = 1, $CHCl_3$), Diastereomerenverhältnis: (1R, 1′R) : (1R, 1′S) = I : II = 60:40

IR (Film): 3500 (O-H), 2980 (C-H), 2900 (C-H), 1750 (C=O), 1370 (C-H), 1240 (C-O), 1130, 1040, 980 $cm^{-1}$

$^1$H-NMR (200 MHz, $CDCl_3$): δ = 1.22 (t, $J_{2'',1''}$ = 7,0 Hz; 1,8 H, 2″-$H_3$, I), 1.24 (t, $J_{2'',1''}$ = 7,0 Hz; 1,2 H, 2″-$H_3$, II), 1.78-2.02 (m; 4 H, 2-$H_2$, 3-$H_2$), 2.06, 2.10 (2xs, 6 H, OAc), 2,38 (s(br)); 1 H, OH, $D_2O$-Austausch), 3.52 (dq; $J_{1'',2''}$ = 7,0 Hz, $J_{1'',1'''}$ = 9,5 Hz; 0,4 H, 1″-H, II), 3.55 (dq, $J_{1'',2''}$ = 7,0 Hz, $J_{1'',1'''}$ = 9,5 Hz; 0,6 H, 1″-H, I), 3.64-3.92 (m; 3 H, 1‴-H, 3′-$H_2$), 3.92-4.32 (m; 3 H, 6-$H_2$, 5-H), 4.66-4.82 (m; 1 H, 4-H), 4.87 (dd, $J_{1',2'}$ = 6,0 Hz, $J_{1',2'}$ = 4,5 Hz; 0,4 H, 1′-H, II), 4.95 (t, $J_{1',2'}$ = 5,5 Hz; 0,6 H, 1′-H, I), 5.01 (s(br); 0,4 H, 1-H, II), 5.16 (s(br); 0,6 H, 1-H, I)

$^{13}$C-NMR: (50 MHz, $CDCl_3$): δ = 15.26 (C-2″, II), 15.29 (C-2″, I), 20.77. 21.06 (OAc), 27.76 (C-3, II), 23.94 (C-3, I), 28.52 (C-2, I), 29.24 (C-2, II), 37.14 (C-2′), 58.62 (C-3′, I), 58.71 (C-3′, II), 62.87, 63.28, 63.91 (C-6, C-1″), 67.78, 69.33 (C-4, C-5, II), 67.83, 69.22 (C-4, C-5, I), 93.09 (C-1, II), 93.70 (C-1, I), 99.93 (C-1′, I), 102.20 (C-1′, II), 170.05, 170.91 (C=O)

MS (70 eV): m/e = 216 (5 %, M + $H^+$-Aglycon), 215 (47 %, M-Aglycon), 155 (11 %, M-Aglycon-HOAc), 103 (100 %, $CH(OC_2H_5)CH_2CH_2OH$), 95 (16 %), 75 (37 %, HO-CH-$CH_2$-$CH_2$OH), 43 (91 %, $CH_3CO$)

| | | | | | |
|---|---|---|---|---|---|
| $C_{15}H_{26}O_8$ (334.4) | Ber. | C | 53.88 | H | 7.84 |
| | Gef. | C | 54.03 | H | 7.92 |

Beispiel 14

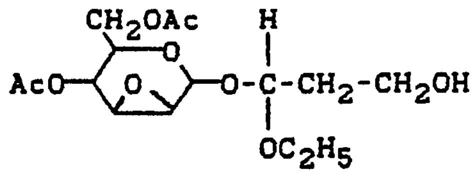

(1'RS)-1'-Ethoxy-3'-hydroxy-propyl-4,6-di-O-acetyl-2,3-anhydro-α-D-mannopyranosid

619 mg (1'RS)-1'-Ethoxy-3'-phenylmethoxy-propyl-4,6-di-O-acetyl-2,3-anhydro-α-D-mannopyranosid (Beispiel 9) (1.41 mmol) wurden nach der AAV4 umgesetzt. Nach 0.5 h wurde aufgearbeitet und an 10 g Kieselgel (LS 1a) chromatographiert. Man erhielt 422 mg (86 %) eines farblosen Öls. $R_F$ = 0.19 (LS 1a) $[\alpha]_D^{20}$ = 94.8° (c = 1, $CHCl_3$), (1R, 1'R):(1R,1'S) = I:II = 60 : 40

IR (Film): 3500 (O-H), 2980, 2940 (C-H), 1740 (C=O), 1370 (C-H), 1230 (C-O), 1130, 1040, 970 $cm^{-1}$
$^1$H-NMR (200 MHz, $CDCl_3$): δ = 1.26 (t, $J_{2'',1''}$ = 7,0 Hz; 3 H, 2''-$H_3$), 1.93-2.05 (mc; 2 H, 2'-$H_2$), 2.07, 2.08, 2.13 (3xs; 6 H, OAc), 2.23 (s(br); 1 H, OH, $D_2O$-Austausch), 3.07 (dd, $J_{3,2}$ = 3.5 Hz, $J_{3,4}$ = 1,0 Hz; 0,4 H, 3-H, II), 3.16 (dd, $J_{3,2}$ = 3,5 Hz, $J_{3,4}$ = 1,0 Hz; 0,6 H, 3-H, I), 3,26 (d, $J_{2,3}$ = 3,5 Hz; 1 H, 2-H), 3.57, 3.59 (2xdq, $J_{1'',2''}$ = 7,0 Hz, $J_{1'',1'''}$ = 9,5 Hz; 1 H, 1''-H), 3.69-4.20 (m; 6-$H_2$, 5-H, 3'-$H_2$, 1'''-H), 4.84 (dd, $J_{4,3}$ = 1.0 Hz, $J_{4,5}$ = 10.0 Hz; 0,6 H, 4-H, I), 4.88 (dd, $J_{4,3}$ = 1,0 Hz, $J_{4,5}$ = 10.0 Hz; 1 H, 4-H, II), 4.96 (dd, $J_{1',2'}$ = 6,0 Hz, $J_{1',2'}$ = 5,0 Hz; 0,4 H, 1'-H, II), 5.03 (t, $J_{1',2'}$ = 5,0 Hz; 0,6 H, 1'-H, I), 5.30 (s; 0,4 H, 1-H, II), 5.34 (s; 0,6 H, 1-H, I)
$^{13}$-C-NMR (50 MHz; $CDCl_3$): δ = 15.16 (C-2'', II) 15.28 (C-2'', I), 20.66, 20.70, 20.79 (OAc), 37.14 (C-2'), 48.11, 53.28 (C-2, C-3, I), 49.38, 53.11 (C-2, C-3, II), 58.43 (C-3', I), 58.55 (C-3', II), 62.85, 65.13 (C-4, C-5, I), 63.09, 65.22 (C-4, C-5, II), 63.14, 64.11 (C-6, C-1''), 90.82 (C-1, II), 92.12 (C-1, I), 100.63 (C-1', I), 102.43 (C-1', II), 169.46, 170.51, 170.56 (C=O)
MS (200 eV, DCI, $NH_3$): m/e = 366 (100 %, M + $NH_4^+$)

| | | | |
|---|---|---|---|
| $C_{15}H_{24}O_9$ (348.4) | Ber. | C 51.72 | H 6.94 |
| | Gef. | C 51.53 | H 7.04 |

Beispiel 15

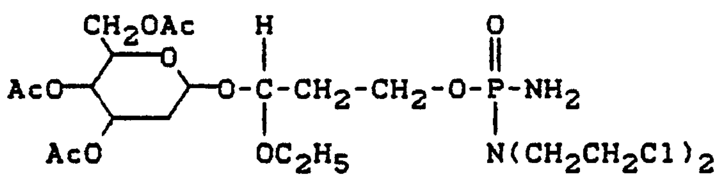

(3'RS)-1'-O-(3'-Ethoxy-3'-(3,4,6-tri-O-acetyl-2-desoxy-α-D-arabino-hexopyranosyloxy))-propyl-N,N-bis-(2-chlorethyl)-phosphorsäurediamidat

370 mg der Verbindung aus Beispiel 10 (0.94 mmol) wurden nach der AAV5 mit N,N-Bis-(2-chlorethyl)-phosphorsäureamiddichlorid umgesetzt. Nach 48 h hatte das Edukt quantitativ abreagiert. $R_F$ = 0.30 (LS 1) (Zwi schenprodukt). Ammoniak wurde 3 h lang eingeleitet. Chromatographie an 30 g Kieselgel (LS 3b) erbrachten 235 mg (42 %) eines farblosen Öls. $R_F$ = 0.30 (LS 3b). $[\alpha]_D^{20}$ = +62.4° (c = 1, $CHCl_3$). Das Diastereomerenverhältnis betrug 1:1 : 3:3.

IR (Film): 3700-3100 (O-H, N-H), 2980, 2900 (C-H), 1740 (C=O), 1370 (C-H), 1240 (C-O), 1140, 1100 (P-N), 1040, 980, 740 $cm^{-1}$ (C-Cl)

$^1$H-NMR (200 MHz, $CDCl_3$): δ = 1.21 (t, $J_{2'',1''}$ = 7.0 Hz; 3 H, 2'''-$H_3$), 1.76-2.08 (m; 3 H, 2''-$H_a$, 2'-$H_2$), 2.03, 2.06, 2.10 (3xs; 9 H, OAc), 2.18-2.34 (m; 1 H, 2''-$H_e$), 2.78-2.96 (m; 2 H, $NH_2$ ($D_2O$-Austausch)), 3.36-3.84 (m; 10 H, 1'''-$H_2$, 2 x N-$CH_2$-$CH_2$), 3.96-4.24 (m; 4 H, 6''-H, 5''-H, 3'-$H_2$), 4.31 (2xdd, $J_{6''',5''}$ = 5.0 Hz, $J_{6''',6''}$ = 11.5 Hz; 1 H, 6'''-H), 4.79, 4.80. 4.85, 5.86 (4xt, $J_{3',2'}$ = 5,5 Hz; 1 H, 3'-H), 5.02, 5.04 (2xt, $J_{4,3}$ = $J_{4,5}$ = 10.0 Hz; 1 H, 4''-H), 5.24-5.30 (m; 2 H, 1''-H, 3''-H)

$C_{21}H_{37}Cl_2N_2O_{11}P$ (595,4)

| | | | |
|---|---|---|---|
| Ber. | C 42,46 | H 6.26 |
| Gef. | C 42.54 | H 6.43 |

Beispiel 16

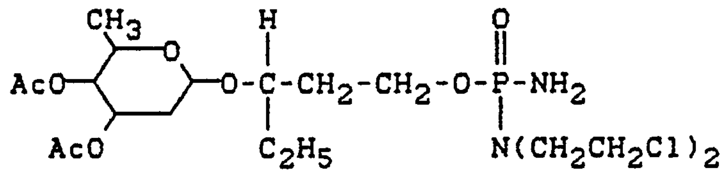

(3'RS)-1'-O-(3'-Ethoxy-3'-(3,4-di-O-acetyl-2,6-didesoxy-α-L-arabino-hexopyranosyloxy))-propyl-N,N-bis-(2-chlorethyl)-phosphorsäurediamidat

367 mg der Verbindung aus Beispiel 11 (1.10 mmol) wurden nach der AAV5 mit N,N-Bis-(2-chlorethyl)-phosphorsäureamid-dichlorid umgesetzt. Nach 24 h war der Umsatz vollständig. $R_F$ = 0.50 (LS 1) (Zwischenprodukt). Es wurde anschließend 5 h lang Ammoniak eingeleitet. Chromatographie an 40 g Kieselgel (LS 5a) ergaben 314 mg (53 %) Produkt. $R_F$ = 0.42 (LS 5a). $[\alpha]_D^{20}$ = -79.7° (c = 1, $CHCl_3$). Diastereomerenverhältnis = 1:1,5:4,5:5

IR (KBr): 3650-3100 (NH), 2980 (C-H), 1740, 1730 (C=O), 1370 (C-H), 1260, 1240 (P=O, C-O), 1140, 1040, 980 $cm^{-1}$.

$^1$H-NMR (200 MHz, $CDCl_3$): δ = 1.17, 1.19 (2xd, $J_{6'',5''}$ = 6.0 Hz; 3 H, 6''-$H_3$), 1.21 (t, $J_{2''',1'''}$ = 7.0 Hz; 3 H, 2'''-$H_3$), 1.70-194 (m; 1 H, 2''-$H_a$), 1.95-2.10 (m; 2 H, 2'-$H_2$), 2.01, 2.05 (2xs; 6 H, OAc), 2.15-2.31 (mc; 1 H, 2''-$H_e$), 2.80-2.94 (m; 2 H, $NH_2$, $D_2O$-Austausch), 3.36-3.78 (m; 10 H, 1'''-H, 1''''-H, 2x$CH_2$-N, 2x$CH_2$-Cl), 3.78-4.25 (m; 3 H, 5''-H, 1'-$H_2$), 4.75, 4.76 (2xt, $J_{4'',3''}$ = 9,5 Hz; 1 H, 4''-H), 4.85. 4.86 (2xt, $J_{3',2'}$ = 5.0 Hz, $J_{3',2'}$ = 5,5 Hz; 1 H, 3'-H), 5.13-5.23 (m; 1 H, 1''-H), 5.17-5.34 (m; 1 H, 3''-H)

$^{13}$C-NMR (50.3 MHz, $CDCl_3$): δ = 15.21, 15.28 (C-2'''), 17.59 (C-6''), 20.86, 21.01 (OAc), 35.02, 35.17, 35.25, 35.30, 25.39, 35.61, 35.68, 35.73 (C-2'', C-2'), 42.51 ($CH_2$-Cl),49.28 (d, $J_{PNC}$ = 4.9 Hz; $CH_2$-N), 61.28, 61.37, 61.46, 61.55, 62.13, 62.17, 63.38, 63.57 (C-1''', C-1'), 66,30, 66.41, 66.44, 68.84, 69.05 (C-3'', C-5''), 74.54, 74.60 (C-4''), 93.36, 93.72, 93.80 (C-1''), 97.56, 97.63, 100.45, 100.73 (C-1'), 170.15, 170.21, 170.28, 170.32 (C=O)

MS (200 eV, DCI, $NH_3$): m/e = 556, 554 (88 %, 100 %, M + $NH_4^+$), 307, 305 (19 %, 56 %, Aglycon$^+$)

| | | | |
|---|---|---|---|
| $C_{19}H_{35}Cl_2N_2O_9P$ (537.4) | Ber. | C 42.47 | H 6.57 |
| | Gef. | C 42.52 | H 6.43 |

Beispiel 17

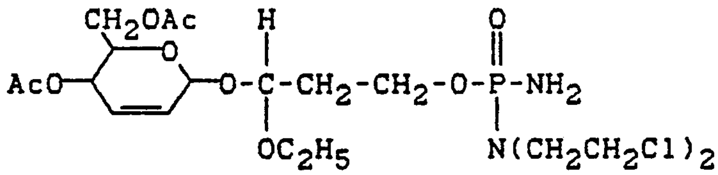

(3'RS)-1'-O-(3'-Ethoxy-3'-(4,6-di-O-acetyl-2,3-di-desoxy-D-erythro-hex-2-enopyranosyloxy))-propyl-N,N-bis-(2-chlorethyl)-phosphorsäurediamidat

300 mg der Verbindung aus Beispiel 12 (0,90 mmol) wurden nach der AAV5 mit N,N-Bis-(2-chlorethyl)-phosphorsäureamid-dichlorid bei Raumtemperatur umgesetzt. Nach 72h war der Umsatz vollständig. $R_F$ = 0.43 (LS 1a) (Zwischenprodukt). Es wurde 4h Ammoniak eingeleitet. Chromatographie an 20 g Kieselgel (LS 3b) ergaben 278 mg (58 %) Produkt. $R_F$ = 0.24 (LS 3b). $[\alpha]_D^{20}$ = +69.4 (c = 0,5, $CHCl_3$). Das Diasteromerenverhältnis betrug: (1RS, 3'R,1''R):(1RS,3'S,1''R) = I:II = (2.5 : 2.5):(1:1)

IR (Film): 3400, 3240, 3100 (O-H, N-H), 2980, 2940, 2900 (C-H), 1740 (C=O), 1570, 1370 (C-H), 1230 (C-O), 1140, 1100, 1040, 980, 750 $cm^{-1}$ (C-Cl)

$^1H$-NMR (200 MHz, $CDCl_3$): δ = 1.23 (t(br), $J_{2''',1'''}$ = 7,0 Hz; 3 H, 2'''-$H_3$), 1.95-2.10 (m; 2 H, 2'-$H_2$), 2.09, 2.11 (2xs, 6 H, OAc), 2.50-2.90 (s(br); 2 H, $NH_2$ ($D_2O$-Austausch)), 3.36-3.88 (m; 10 H, 1'''-$H_2$, N-$CH_2$, $CH_2$-Cl), 3.98-4.35 (m; 5 H, 1'-$H_2$, 5''-H, 6''-$H_2$), 4.86, 4.87 (2xt, $J_{3',2'}$ = 5.5 Hz; 0,3 H, 3'-H, II), 4.96, 4.97 (2xt, $J_{3',2'}$ = 5.5 Hz; 0.7 H, 3'-H, I), 5.25-5.48 (m; 2 H, 1''-H, 4''-H), 5.74-6.01 (m; 2 H, 2''-H, 3''-H)

$^{13}C$-NMR (50 MHz, $CDCl_3$); δ = 15.15 (C-2''', II), 15.26 (C-2''', I), 20.79, 20.95 (OAc), 35.75, 35.89 (C-2'), 42.50 (C-Cl), 49.21, 49.30, 49.37 (C-N), 61.41, 61.51, 61.61, 61.70, 62.30, 62.51, 62.74, 62.88, 63.05, 63.56 (C-1''', C-6'', C-1'), 64.96, 67.42 (C-4'', C-5'', II), 66.12, 67.14 (C-4'', C-5'', I), 90.09, 90.25 (C-1'', II), 91.61, 91.79, (C-1'', I), 99.13 (C-3', I), 100.28, 100.32 (C-3', II), 127.10, 127.18, 129.28, 129.39 (C-2'', C-3'', I), 127,67, 128,90, 128.93 (C-2'', C-3'', II), 170.05, 170.55, 170.63 (C=O)

MS (200 eV, DCI $NH_3$): m/e = 554, 552 (18 %, 26 %, M + $NH_4^+$), 248 (100 %), 161, 159 (62 %, 95 %)

| | | | | | |
|---|---|---|---|---|---|
| $C_{19}H_{33}Cl_2N_2O_9P$ (535.4) | Ber. | C | 42.63 | H | 6.21 |
| | Gef. | C | 43.02 | H | 6.39 |

Beispiel 18

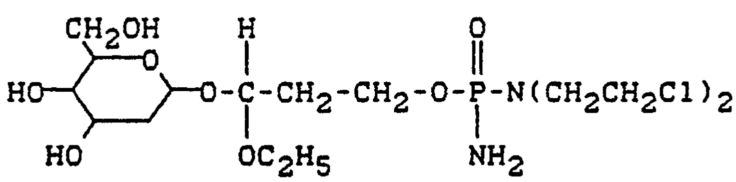

Beispiel 18 kann aus dem Beispiel 15 nach der Arbeitsvorschrift 6 (AAV6) dargestellt werden.

Beispiel 19

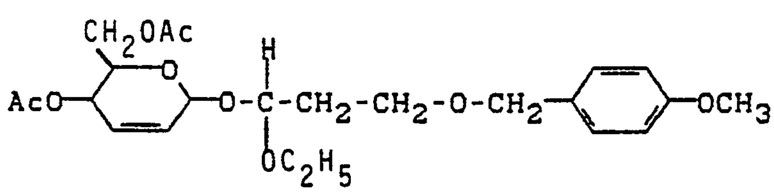

(1'RS)-1'-Ethoxy-3'-(4-methoxyphenyl)-methoxypropyl-4,6-di-O-acetyl-2,3-di-desoxy-D-erythro-hex-2-enopyranosid

300 mg (0,99 mmol) von Beispiel 4 wurden nach der AAV2 bei -90 °C mit 1,2-Diethoxy-3-(4-methoxyphenyl)-methoxypropan (A) und 3-(4-Methoxyphenyl)-methoxy-propanol (B) in Dichlormethan umgesetzt. Nach 24 h gab man die halbe Menge an A, B und Trimethylsilyltriglat mit einer Spritze hinzu. Nach 30 h wurde aufgearbeitet und an 40 g Kieselgel chromatographiert. Man erhielt 368 mg (82 %) eines farblosen Öls. $R_F$ = 0,24 (LS 2e). $[\alpha]_D^{20}$ = + 88° (c = 0,5, $CHCl_3$). Diastereomerenverhältnis: (1R, 1'R) : (1R, 1'S) = I : II = 60 : 40.

IR (Film): 2980, 2940, 2900 (C-H), 1740 (C=O), 1615, 1515 (C=C), 1370 (C-H), 1240 (C-O), 1100, 1040, 820 $cm^{-1}$

$^1$H-NMR (200 MHz, $CDCl_3$): δ = 1,20 (t, $J_{2'',1''}$ = 7,0 Hz; 3 H, 2''-$H_3$, I), 1,90-2,11 (m; 2 H, 2'-$H_2$), 2,04, 2,07, 2,08, 2,09 (4xs; 6 H, OAc), 3,41-3,87 (m; 4 H, 1''-$H_2$, 3'-$H_2$), 3,80 (s; 3 H, $OCH_3$), 4,00-4,30 (m; 3H, 5-H, 6-$H_2$), 4,42 (s(br); 2 H, $PhCH_2O$), 4,88 (t, $J_{1',2'}$ = 5,5 Hz; 0,4 H, 1'-H, II), 4,98 (t, $J_{1',2'}$ = 5,5 Hz; 0,6 H, 1'-H, I), 5,16-5,36 (m; 2 H, 1-H, 4-H), 5,64-5,99 (m; 2 H, 2-H, 3-H), 6.82-6.91 (m; 2 H, Ph-H), 7,19-729 (m; 2 H, Ph-H)

$^{13}$C-NMR (50 MHz, $CDCl_3$): δ = 15,14 (C-2'', II), 15,30 (C-2'', I), 20,57, 20,65, 20,83 (OAc), 35,14 (C-2', I), 35,47 (C-2',II), 55.08 ($OCH_3$), 62,33, 62,88, 65,75 (C-6, C-3', C-1''), 63,05, 63,48, 65,58 (C-6, C-3', C-1'', II), 64,97, 67,27 (C-4, C-5, II), 65,15, 67,12 (C-4, C-5, I), 72,54 ($PhCH_2O$), 90,42 (C-1, II), 91,03 (C-1, I), 99,30 (C-1', I), 101,30 (C-1', II), 113,59 (C-2 arom., C-6 arom. C-6 arom.), 127,47, 127,58, 127,92, 128,55, 128,99, 129,10, 129,16, 129,33 (C-2, C-3, C-3 arom., C-5 arom.), 130,22, 130,29, 130,40, 130,40, 130,45 (C-4 arom.), 159.00, 159.05 (C-1 arom.), 169,90, 170,34, 170,40 (C=O)

Ms (200 eV, DCI, $NH_3$): m/e = 470 (100 %, M + $NH_4^+$)

| | | | |
|---|---|---|---|
| $C_{23}H_{32}O_9$ (452,5) | Ber. | C 61.05 | H 7,13 |
| | Gef. | C 60,69 | H 7,04 |

Beispiel 20

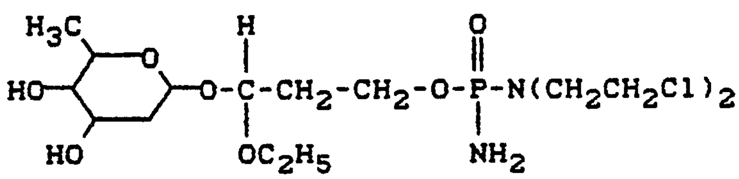

Beispiel 20 kann aus Beispiel 16 nach der Arbeitsvorschrift 6 (AAV6) dargestellt werden.

Beispiel 21

(3'-RS)-1'-O-(3'-Ethoxy-3'-(2,3-di-desoxy-D-erythrohex-2-enopyranosyl-oxy)-propyl-N,N-bis-(2-chlorethyl)-phosphorsäurediamidat

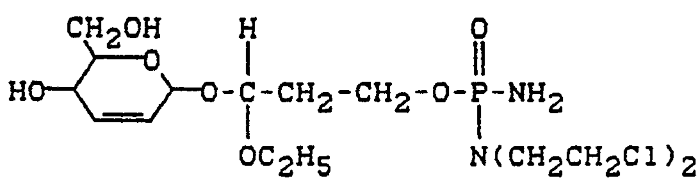

Beispiel 21 kann aus Beispiel 17 nach Arbeitsvorschrift 6 (AAV6) dargestellt werden.

## Ansprüche

1. Verbindungen entsprechend der allgemeinen Formel (I)

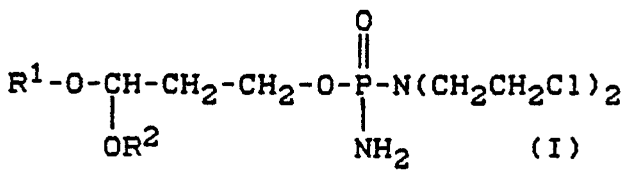

(I)

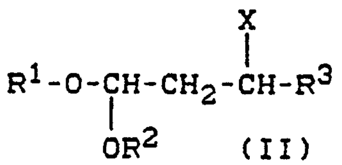

(II)

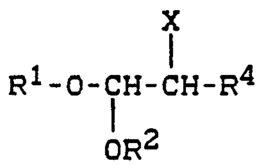

(III)

in welcher
$R^1$ für gesättigtes, ungesättigtes oder anhydridisches Desoxypentosyl, Desoxyhexosyl oder Didesoxyhexosyl steht,
wobei
die OH-Funktionen des Zuckers gegebenenfalls geschützt sind,
und
$R^2$ für geradkettiges oder verzweigtes auch heterosubstituiertes Alkyl mit bis zu zehn Kohlenstoffatomen steht und
$R^3$ für geradkettiges oder verzweigtes auch heterosubstituiertes Alkyl mit bis zu zehn Kohlenstoffatomen und
X für Halogene und
$R^4$ für geradkettiges oder verzweigtes, auch heterosubstituiertes Alkyl mit bis zu zehn Kohlenstoffatomen steht.

2. Verbindungen der allgemeinen Formeln (I), (II) und (III) gemäß Anspruch 1
in welcher
$R^1$ für gesättigtes, ungesättigtes oder anhydridisches Desoxypentosyl, Desoxyhexosyl oder Didesoxyhexosyl steht,
wobei
die OH-Funktionen des Zuckers gegebenenfalls geschützt sind,
und
$R^2$ für geradkettiges oder verzweigtes auch heterosubstituiertes Alkyl mit bis zu zehn Kohlenstoffatomen steht und
$R^3$ für geradkettiges oder verzweigtes auch heterosubstituiertes Alkyl mit bis zu zehn Kohlenstoffatomen und
X für Halogene und
$R^4$ für geradkettiges oder verzweigtes, auch heterosubstituiertes Alkyl mit bis zu zehn Kohlenstoffatomen steht.

3. Verbindungen der allgemeinen Formeln (I), (II) und (III) gemäß Anspruch 1
in welcher
$R^1$ für gesättigtes, ungesättigtes oder anhydridisches 2-Desoxyhexopyranosyl, 2,6-Didesoxyhexopyranosyl, 2-Desoxy-pentofuranosyl, 2,3-Didesoxyhexopyranosyl oder 2,3-Anhydropentofuranosyl steht,
wobei
die OH-Funktionen des Zuckers gegebenenfalls geschützt sind,
und
$R^2$ für geradkettiges oder verzweigtes Alkyl mit bis zu 6 Kohlenstoffatomen und
$R^3$ und $R^4$ für Wasserstoffatomen steht.
Besonders bevorzugt sind die Verbindungen der allgemeinen Formel (I), (II) und (III)
in welcher

$R^1$ für 2-Desoxy-α-D-arabino-hexo-pyranosyl, 2-Desoxy-D-erythro-pento-pyranoxyl, 2,6-Didesoxy-α-L-arabino-hexo-pyranosyl, 2,3-Anhydro-α-D-manno-pyranosyl oder 2,3-Didesoxy-D-erythro-hex-2-eno-pyranosyl steht,

4. Verbindungen der allgemeinen Formel (I) gemäß Anspruch 1,
in welcher
$R^1$ für gesättigtes, ungesättigtes oder anhydridisches 2-Desoxyhexopyranosyl, 2,6-Didesoxyhexopyranosyl, 2-Desoxy-pentofuranosyl, 2,3-Didesoxyhexopyranosyl oder 2,3-Anhydropentofuranosyl steht, welches über den Sauerstoff am anomeren Kohlenstoffatom glykosdisch verknüpft ist,
wobei
die OH-Funktionen gegebenenfalls geschützt sind,
und
$R^2$ für geradkettiges der verzweigtes Alkyl mit bis zu 6 Kohlenstoffatomen steht.

5. Verbindungen der allgemeinen Formel (I) gemäß Anspruch 1,
in welcher
$R^1$ für 2-Desoxy-α-D-arabino-hexo-pyranosyl, 2-Desoxy-3,5-di-erythro-pento-pyranoxyl, 2,6-Didesoxy-α-L-arabino-hexo-pyranoxyl, 2,3-Anhydro-α-D-manno-pyranosyl oder 2,3-Didesoxy-D-erythro-hex-2-eno-pyranosyl steht, welches über den Sauerstoff am anomeren Kohlenstoffatom glykosidisch verknüpft ist,
wobei
die OH-Funktion gegebenenfalls geschützt sind und
$R^2$ für geradkettiges Alkyl mit bis zu 3 Kohlenstoffatomen steht.

6. Verbindungen der allgemeinen Formel (I)
in welcher
$R^1$ für 2-Desoxy-α-D-arabino-hexo-pyranosyl, 2,6-Di-desoxy-α-L-arabino-hexo-pyranosyl oder 2,3-Didesoxy-D-erythro-Lex-2-eno-pyranosyl steht, welches über den Sauerstoff am anomeren Kohlenstoffatom glykosidisch ist,
wobei
die OH-Funktionen gegebenenfalls geschützt sind
und
$R^2$ für Methyl oder Ethyl steht.

7. Verfahren zur Herstellung der erfindungsgemäßen Verbindungen der allgemeinen Formel (I) gemäß den Ansprüchen 1 bis 4,
dadurch gekennzeichnet,
daß man Verbindungen der allgemeinen Formel (IV)

$$R^1\text{-O-}\underset{\substack{|\\ OR^2}}{CH}\text{-}CH_2\text{-}CH_2\text{-}R^3 \quad (IV)$$

in welcher
$R^1$ und $R^2$ die in den Ansprüchen 1 bis 4 angegebene Bedeutung haben und
$R^3$ für Hydroxy steht
mit
N,N-Bis(2-chlorethyl)-phosphorsäuramid-dichlorid der allgemeinen Formel (V)

$Cl_2PON(CH_2CH_2Cl)_2$ (V)

in Anwesenheit von Basen umsetzt, nachfolgend eine Ammonolyse durchführt und gegebenenfalls die Schutzgruppen abspaltet.

8. Arzneimittel enthaltend eine oder mehrere der Verbindungen gemäß den Ansprüchen 1 bis 6.